(19) Europäisches Patentamt
European Patent Office
Office européen des brevets

(11) Publication number: **0 470 604 A2**

(12) **EUROPEAN PATENT APPLICATION**

(21) Application number: **91113293.4**

(22) Date of filing: **08.08.91**

(51) Int. Cl.[5]: **C12N 15/16**, C07K 13/00, C12N 1/21, C12P 21/02, //(C12N1/21,C12R1:19)

The applicant has filed a statement in accordance with Rule 28 (4) EPC (issue of a sample only to an expert). Accession number(s) of the deposit(s): FERM BP-3054 and 3033.

(30) Priority: **10.08.90 JP 210327/90**

(43) Date of publication of application: **12.02.92 Bulletin 92/07**

(84) Designated Contracting States: **AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Onda, Haruo**
**5-26, Shimotakatsu 4-chome**
**Tsuchiura, IBaraki300(JP)**
Inventor: **Kimura, Chiharu**
**9-7-204, Hanabatake 3-chome**
**Tsukuba, Ibaraki 300-32(JP)**

(74) Representative: **von Kreisler, Alek, Dipl.-Chem. et al**
**Patentanwälte Von Kreisler-Selting-Werner,**
**Deichmannhaus am Hauptbahnhof**
**W-5000 Köln 1(DE)**

(54) **PACAP38, proteine with adenylate cyclase activity.**

(57) Disclosed are (1) a genomic DNA of human PACAP38; (2) a DNA containing a DNA segment coding for human PACAP38; (3) a transformant bearing a DNA containing a DNA segment coding for PACAP38; and (4) a method for preparing a mature PACAP38 protein comprising cultivating the transformant described in the above (3), accumulating the protein in a culture product, and collecting the resulting protein. The DNA makes it possible to screen the chemical substance necessary for production of PACAP and is applied to experimental animals to understand their brain functions, which serves to elucidate human brain functions. Human PACAP38 can also be utilized as therapeutic agents about growth and maintenance of human brain nerves.

EP 0 470 604 A2

## BACKGROUND OF THE INVENTION

The present invention relates to a genomic DNA of human PACAP 38 which is a bioactive peptide derived from testes or brain hypothalami, a DNA containing a DNA sequence coding for the peptide, a transformant bearing the DNA, and a method for preparing a mature PACAP38 peptide in a transformant.

Various hormones secreted by brain hypothalami and hypophyses are well known. Examples thereof include thyrotropin releasing hormone, luteinizing hormone releasing hormone, somatostatin, adrenocorticotropic hormone(ACTH), growth hormone(GH) and prolactin. The action of these hormones has been well studied. A novel peptide consisting of 38 amino acid residues which has the adenylate cyclase activity was discovered in a different hormone derived from sheep hypophyse. The structure thereof was determined and the peptide was named "PACAP38" (EP-A 0 404 652).

The present inventors filed a patent application (Japanese Patent Application No. 1-155791/1990) on cDNA of sheep PACAP38. Further, cDNA of human PACAP38 was cloned from the cDNA library of testes and the amino acid sequence thereof was also determined (Japanese Patent Application No. 1-259924/1990).

The amino acid sequence corresponding to human PACAP38 is the same as that of sheep PACAP38, although there is substitution of some amino acids in the precursors thereof.

In general, when an amino acid sequence such as a bioactive peptide is determined and cDNA is cloned, its expression mechanism is studied to determine the physical conditions under which transcription and translation of the gene take place. Understanding the expression mechanism aids in the development of drugs for inducing the expression of the gene. PACAP38, a peptide consisting of 38 amino acid residues which enhances adenylate cyclase activity, is one such peptide whose expression mechanism was unknown in the prior art.

## SUMMARY OF THE INVENTION

The present inventors have undertaken the study of the mechanism of the expression of the human PACAP38 gene in order to develop methods for inducing the expression. Their study has led to the isolation of genomic DNA of PACAP38 from a human DNA library and the determination of its nucleotide sequence. This has made it possible to induce human PACAP38 through genetic engineering techniques, thus achieving the present invention.

Concurrently, the present inventors have further cloned the gene of human PACAP38, which has been compared with the structure of the previously cloned cDNA of human PACAP38 to determine the structure thereof and to elucidate the relationship between an intron and an exon. At the same time, a promoter region essential for the expression of the gene has been identified.

In accordance with the present invention, there are provided (1) a genome of human PACAP38; (2) a DNA containing a DNA sequence coding for human PACAP38; (3) a transformant bearing a DNA containing a DNA segment coding for PACAP38; and (4) a method for preparing a mature PACAP38 protein comprising cultivating the transformant described in the above (3), accumulating the protein in a culture product, and collecting the resulting protein.

## BRIEF DESCRIPTION OF THE DRAWINGS

Fig. 1 is a simplified restriction enzyme map of a genomic DNA of human PACAP38; and

Fig. 2 [SEQ. ID. NO:1] shows a nucleotide sequence of the genomic DNA of human PACAP38 and an amino acid sequence of a human PACAP38 precursor from which a mature peptide cna be deduced.

## DESCRIPTION OF THE PREFERRED EMBODIMENTS

In the present invention, an expression vector containing a DNA having the nucleotide sequence coding for the precursor protein of human PACAP38 or mature PACAP38 can be prepared, for example, by the following process:

(i) Human cell DNA is extracted and treated with restriction enzyme such as EcoRI or SalI, and is introduced into a phage or a plasmid;

(ii) The recombinant phage or plasmid thus obtained is introduced into an appropriate host cell to produce a transformant;

(iii) After cultivation of the transformant thus obtained, the plasmid or the phage containing the desired DNA is isolated from the transformant by an appropriate method such as hybridization with a DNA probe coding for a portion of PACAP38;

(iv) The desired cloned DNA is cut out from the recombinant DNA; and

(v) The cloned DNA or a portion thereof is ligated downstream from a promoter in a vector suitable for expression, whereby an expression vector can be obtained.

The plasmids into which the DNA is introduced include, for example, pBR322 [Gene 2, 95 (1977)], pBR325 [Gene 4, 121 (1978)], pUC12 [Gene 19, 259 (1982)] and pUC13 [Gene 19, 259 (1982)], each derived from Escherichia coli, and pUB110 derived from Bacillus subtilis [Biochemical and Biophysical Research Communication 112, 678 (1983)]. However, any other plasmid can be used as long as it is replicable and viable in the host. The phage vectors into which the DNA is introduced include, for example, λgt11 [R. Young and R. Davis, Proc. Natl. Acad. Sci., U.S.A. 80, 1194 (1983)] and EMBL3 [Prischauf et al., J. Mol. Biol. 170, 827 (1983)]. However, any other phage vector can be used as long as it is replicable and viable in the host.

Methods for introducing the DNA into the plasmid include, for example, the method described in T. Maniatis et al., Molecular Cloning, p.239, Cold Spring Laboratory, (1982). Methods for introducing the DNA into the phage vector include, for example, the method of T. V. Hyunh et al. [DNA Cloning, A Practical Approach 1, 49 (1985)].

The plasmid thus obtained is introduced into the appropriate host cells such as Escherichia and Bacillus.

Examples of Escherichia described above include Escherichia coli K12DH1 [Proc. Natl. Acad. Sci. U.S.A. 60, 160 (1968)], M103 [Nucleic Acids Research 9, 309 (1981)], JA221 [Journal of Molecular Biology 120, 517, (1978)], HB101 [Journal of Molecular Biology 41, 459 (1969)] and C600 [Genetics 39, 440 (1954)].

Examples of Bacillus described above include Bacillus subtilis MI114 [Gene 24, 255 (1983)] and 207-21 [Journal of Biochemistry 95, 87 (1984)].

Methods for transforming the host with the plasmid include, for example, the calcium chloride method and the calcium chloride/rubidium chloride method described in T. Maniatis et al., Molecular Cloning, p.249, Cold Spring Harbor Laboratory, (1982).

When the phage vector is used, for example, the phage vector can be transduced into proliferated E. coli, using the in vitro packaging method.

A human DNA library can be obtained by the methods described above and the like.

Methods for cloning the PACAP38 DNA from the human DNA library include, for example, the plaque hybridization method [T. Maniatis et al., Molecular Cloning, Cold Spring Harbor Laboratory (1982)] using as a probe an oligonucleotide chemically synthesized reflecting phage vector λgt11 or EMBL and the amino acid sequence of PACAP38, or the cDNA of PACAP38.

The PACAP38 cDNA thus cloned is subcloned to a plasmid such as pBR322, pUC12, pUC13, pUC18, pUC19, pUC118, pUC119 or the like to obtain the human PACAP38 DNA, if necessary.

The nucleotide sequence of the DNA thus obtained is determined, for example, by the Maxam-Gilbert method [A. M. Maxam and W. Gilbert, Proc. Natl. Acad. Sci., U.S.A. 74, 560 (1977)] or the dideoxy method [J. Messing et al., Nucleic Acids Research 9, 309 (1981)], and the existence of the human PACAP38 DNA is confirmed in comparison with the known amino acid sequence.

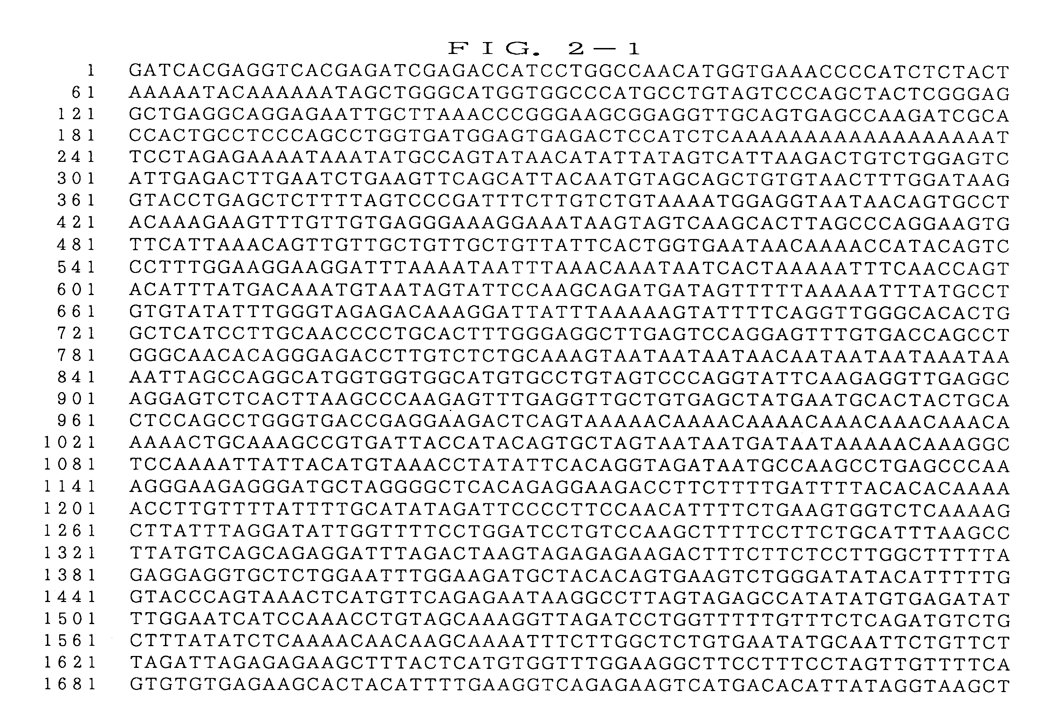

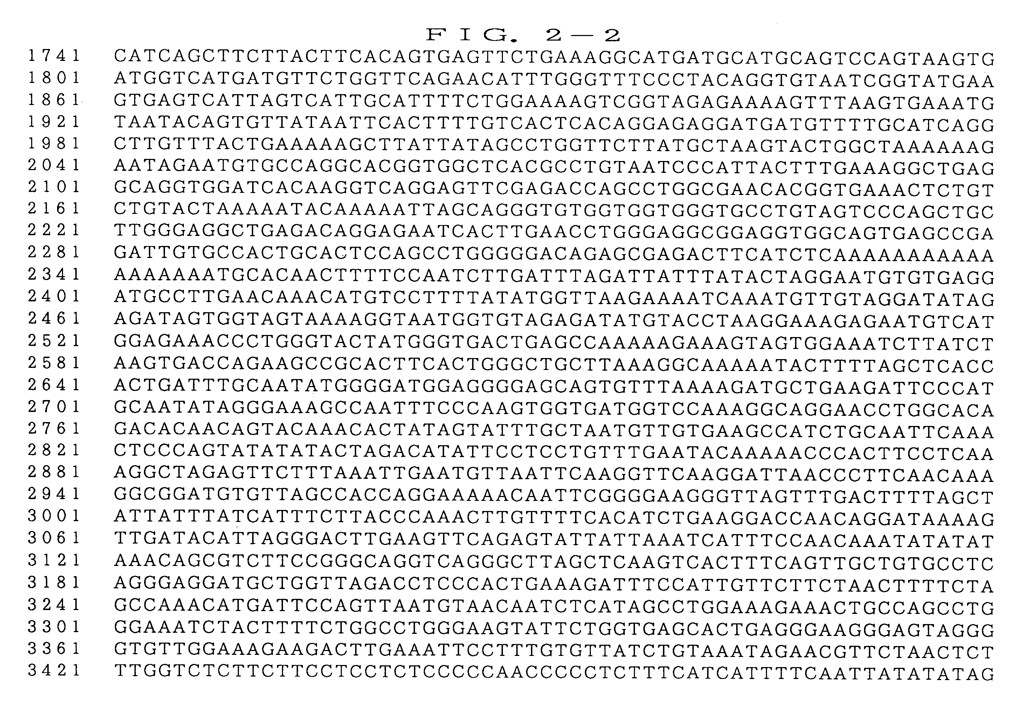

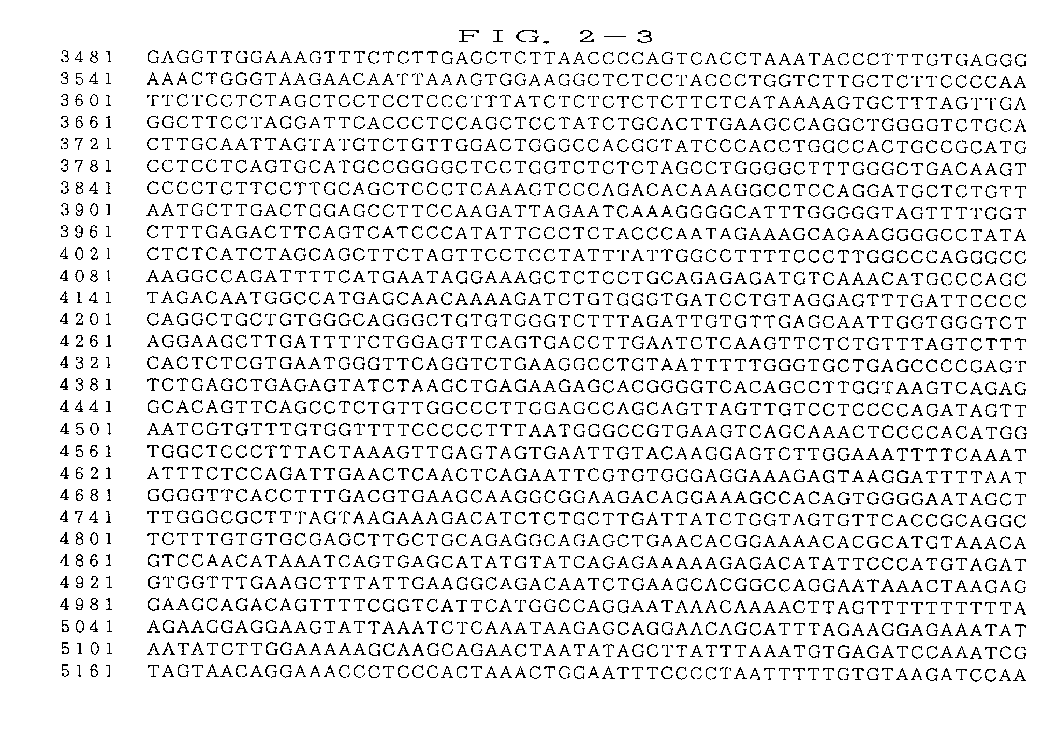

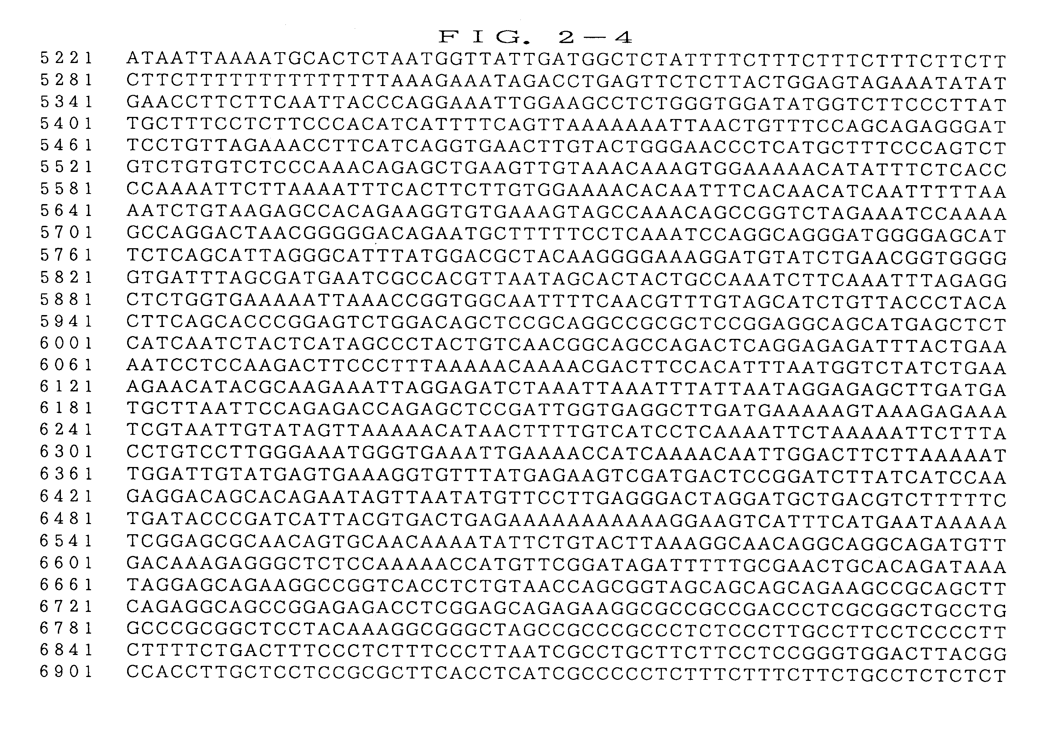

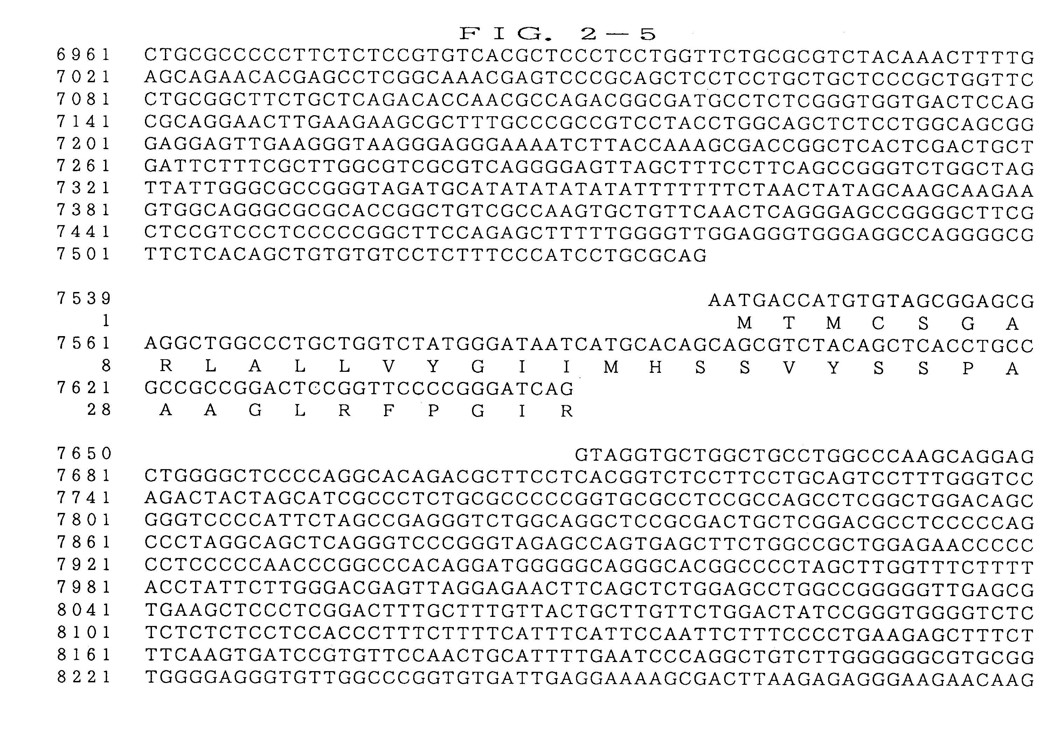

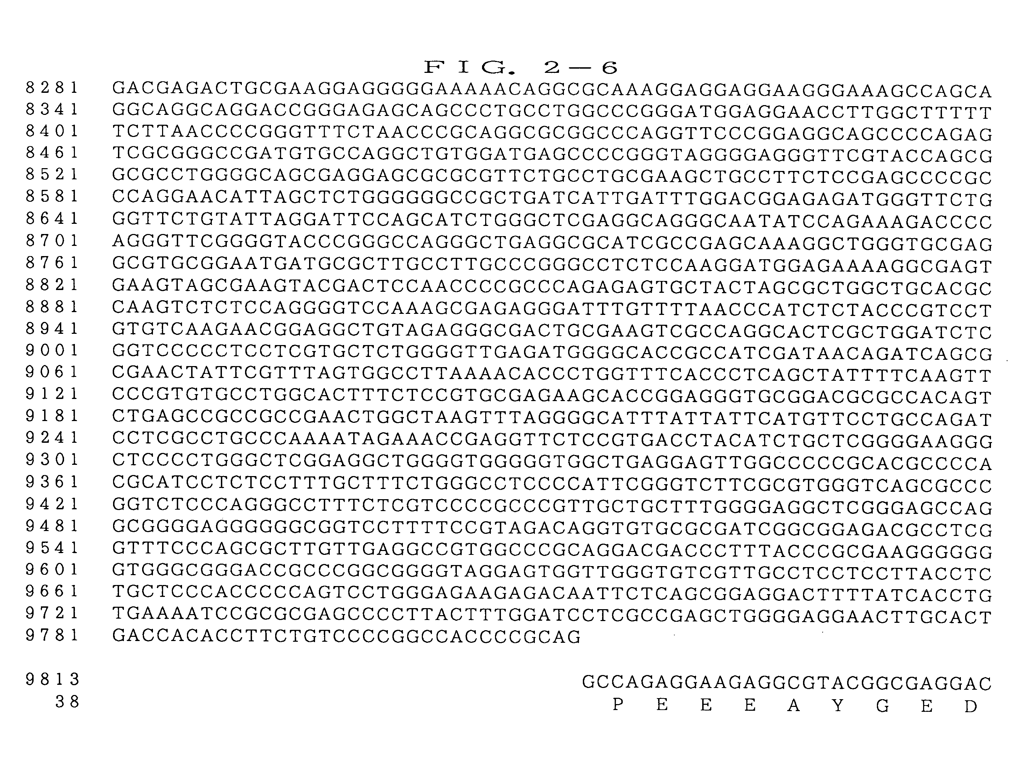

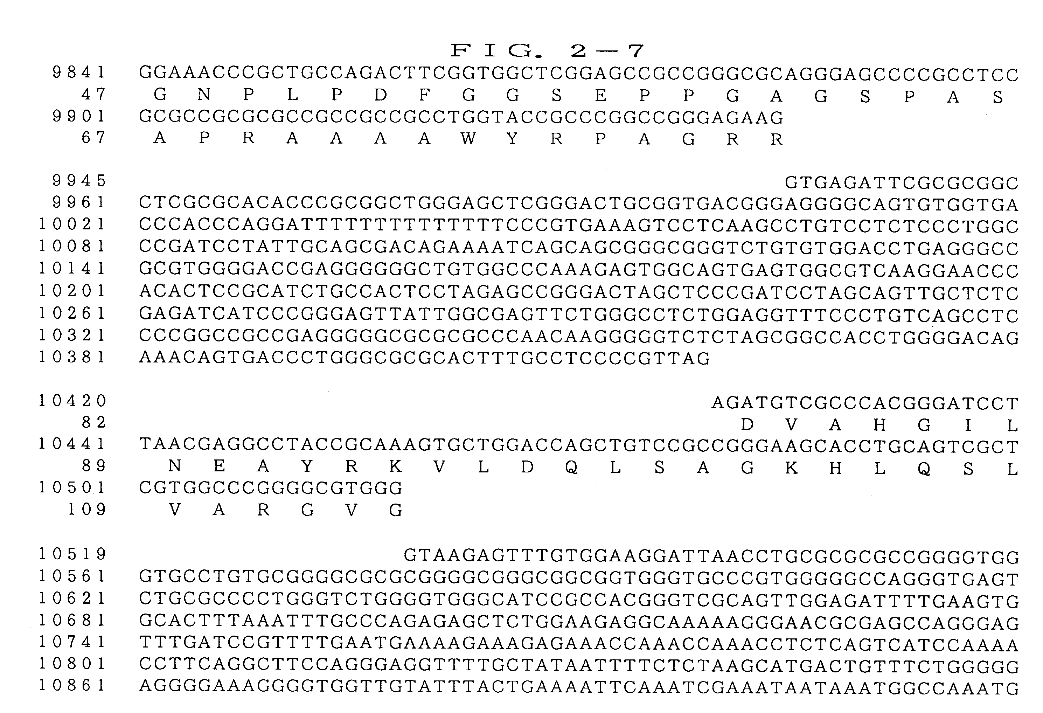

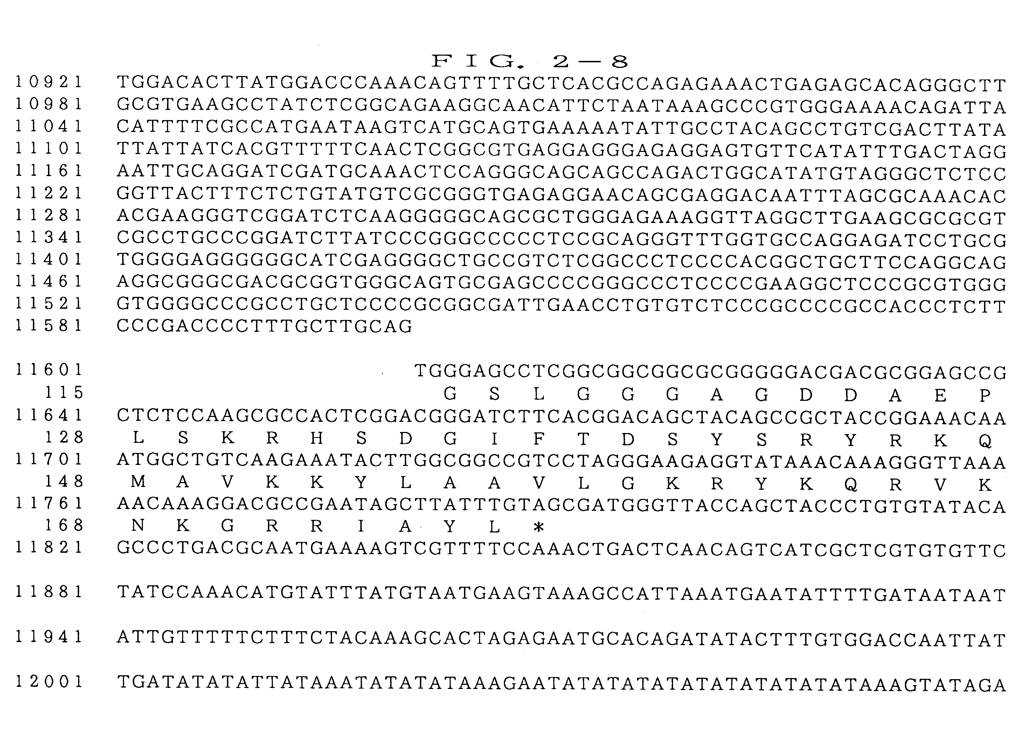

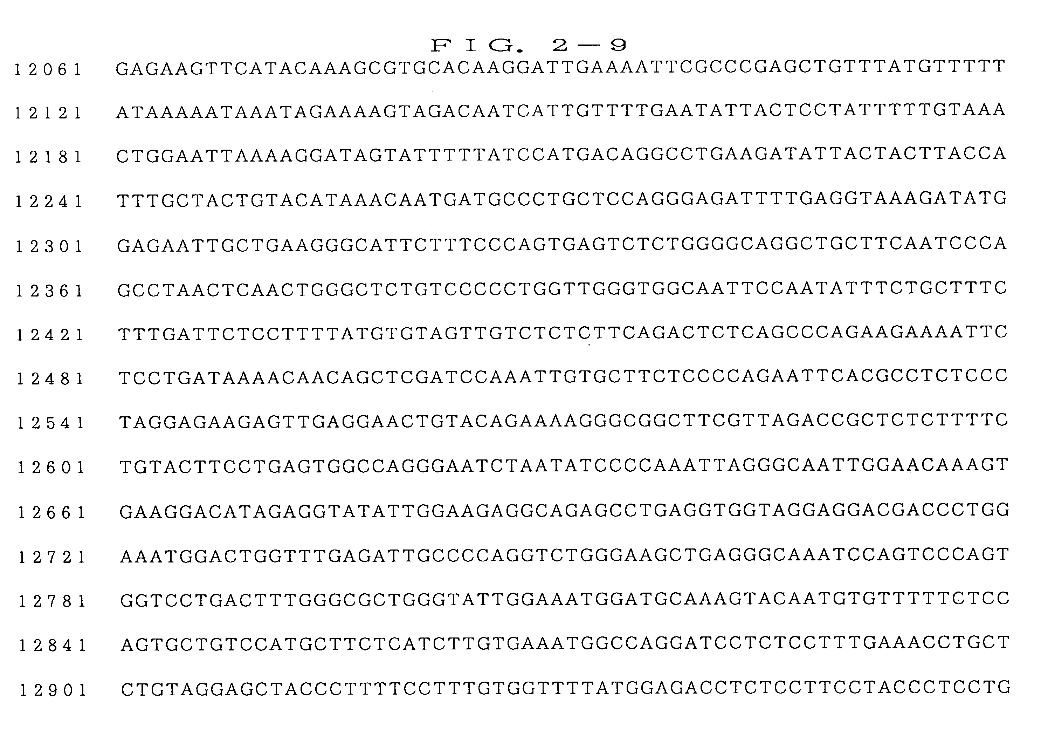

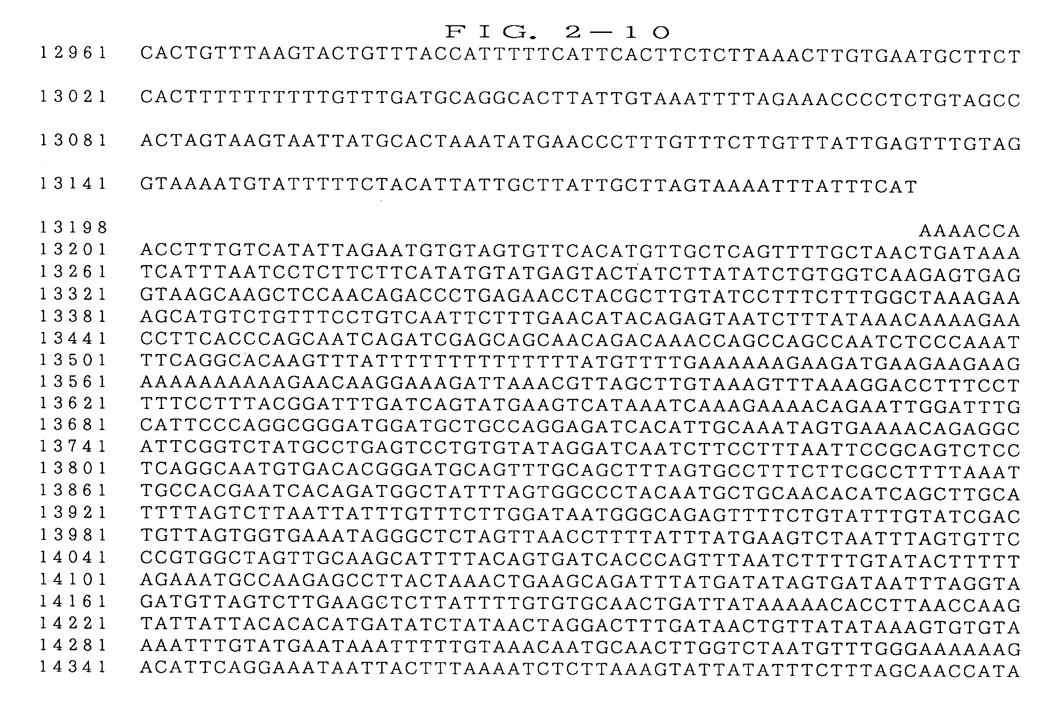

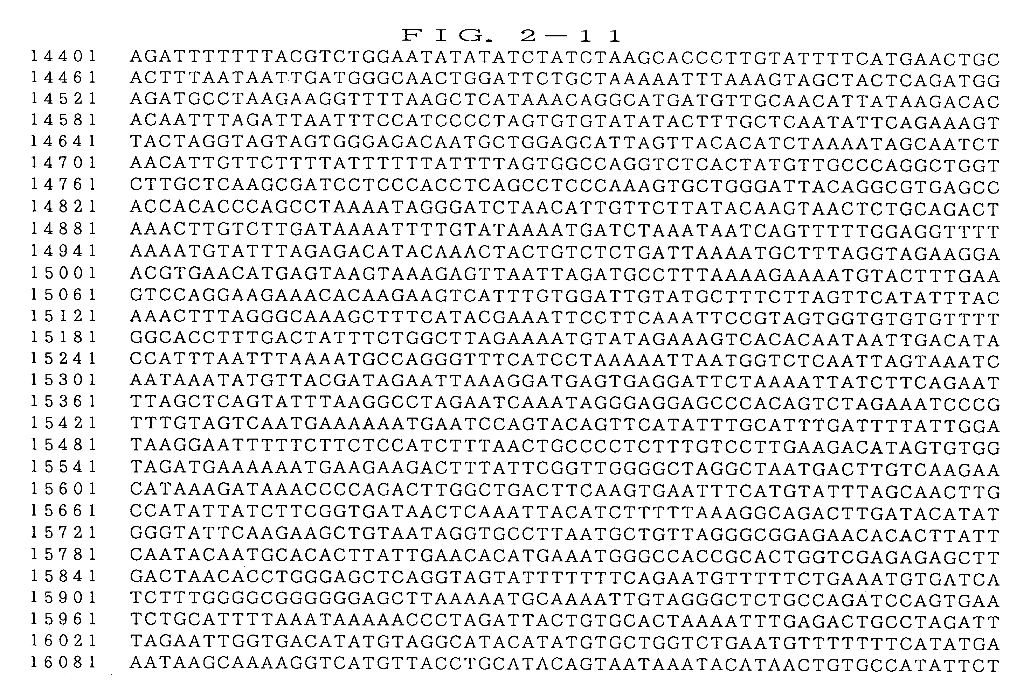

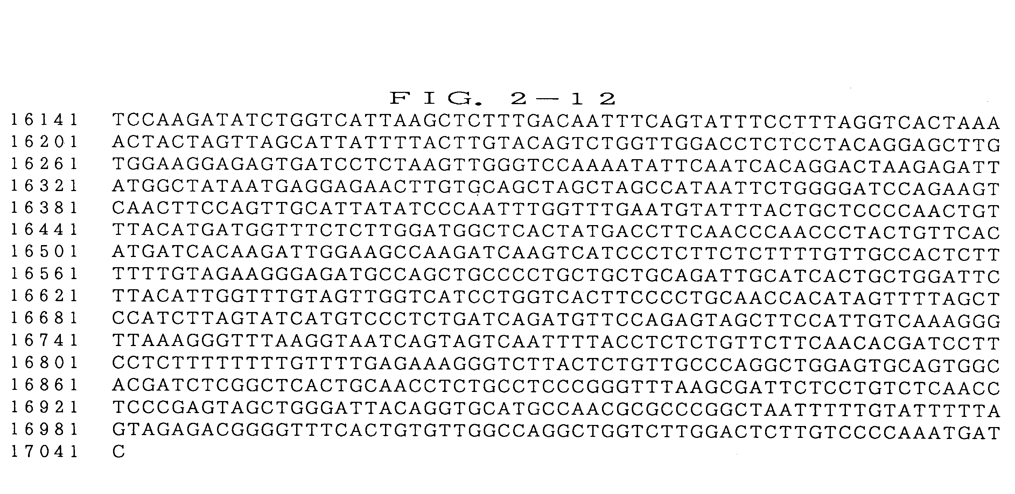

As described above, the DNA (Fig. 2) coding for a portion of the precursor protein of human PACAP38 is obtained.

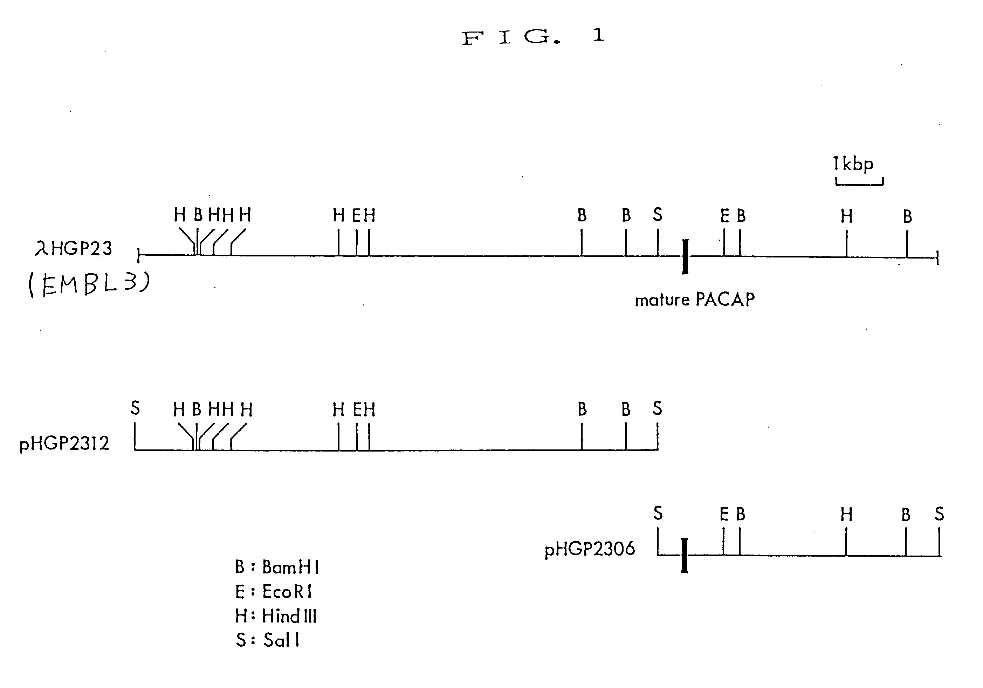

The restriction enzyme fragment map of the DNA coding for the precursor protein of human PACAP38 is shown in Fig. 1. The nucleotide sequence of the DNA determined by the dideoxy method and the amino acid sequence deduced from that nucleotide sequence are shown in Fig. 2.

The DNA coding for the precursor protein of human PACAP38 cloned as described above can be used as is, or cut out by digestion with a restriction enzyme if desired, according to the intended use.

The region intended to be expressed is cut out from the cloned DNA and ligated downstream from the promoter in a vehicle (vector) suitable for expression, whereby the expression vector can be obtained.

The DNA has ATG as a translation initiating codon at the 5'-terminus thereof and may have TAA, TGA or TAG as a translation terminating codon at the 3'-terminus. Further, a consensus sequence reactive to cyclic AMP is observed upstream from ATG of the 5'-terminus. These translation initiating codon and translation terminating codon may be added by use of an appropriate synthetic DNA adaptor such as adaptor containing a translation initiating codon ATG or a translation terminating codon TAA, TAG or TGA.

The vectors include the above plasmids derived from E. coli such as pBR322, pBR325, pUC12 and pUC13, plasmids derived from Bacillus subtilis such as pUB110, pTP5 and pC194, plasmids derived from yeast such as pSH19 and pSH15, bacteriophages such as λ phage, and animal viruses such as retroviruses and vaccinia viruses.

When the host is an animal cell, a SV40-derived promoter, a retrovirus promoter, a metallothionein promoter, a heat shock promoter or the like can be used.

The use of an enhancer such as an SV40-derived enhancer, a retrovirus-derived enhancer, is also effective for expression.

By using a vector containing the DNA coding for the precursor protein of human PACAP38 or the mature peptide PACAP38 thus constructed, the transformant is prepared.

Examples of the animal cells include monkey cell COS-7, Vero, Chinese hamster cell (CHO), mouse L cell and human FL cell.

The transformation of Escherichia described above is conducted, for example, according to the method described in Proc. Natl. Acad. Sci. U.S.A., 69, 2110 (1972), Gene, 17, 107 (1982) or the like.

The transformation of animal cells is carried out, for example, according to the method described in Virology, 52, 456 (1973).

Thus, the transformant transformed with the expression vector containing the DNA coding for the precursor protein of human PACAP38 or the mature peptide (PACAP38) is obtained.

The pH of the medium is preferably about 5 to 8.

When the animal cell transformants are cultivated, examples of the media which can be used include MEM medium containing about 5 to 20% fetal calf serum [Science, 122, 501 (1952)], DMEM medium [Virology, 8, 396 (1959)], RPMI1640 medium [The Journal of the American Medical Association, 199, 519 (1967)] and 199 medium [Proceeding of the Society for the Biological Medicine, 73, 1 (1950)]. The pH is preferably about 6 to 8. The cultivation is usually carried out at about 30 to 40 °C for about 15 to 60 hours, with aeration or agitation if necessary.

In the animal cells transfected with the DNA of the present invention, induction of PACAP is observed by introducing the 5'-region (promoter region) fragment of the genomic DNA of PACAP38 into the animal cell expression vector, whereby the promoter activity of PACAP can be examined. For example, when a recognition sequence of a cyclic AMP response element (CRE) or 12-O-tetradecanoylphorbol-13 acetate (TPA) exists in the promoter region of the genomic DNA, synthesis of mRNA is specifically initiated from the genomic DNA by adding cyclic AMP, a compound similar thereto or TPA to the cells, which results in protein synthesis. Further, using an induction substance (chemical substance) for the PACAP gene, the amount of PACAP produced can be determined, for example, by the use of the sandwich EIA method. Furthermore, by using this experimental system, it is possible to screen the chemical substance (drug) necessary for production of PACAP. Substances showing promise during screening may be given to experimental animals to understand their action, particularly on brain functions, more particularly on brain functions due to hormones. Moreover, the information thus obtained provides information which serves to elucidate human brain functions.

Human PACAP38, including PACAP27, results in an increase in cAMP activity, and therefore can be utilized as therapeutic agents for growth and maintenance of human brain nerves.

There were hereinbefore described in detail the cloning of the DNA coding for human PACAP38, the preparation of the promoter activity expression vectors, the preparation of the transformants thereby, the production of a portion of human PACAP38 and the mature peptides by use of the transformants, and the utility thereof.

When the amino acids are capable of existing as optical isomers, it is understood that the L-forms are represented unless otherwise specified. When nucleotides, amino acids and so on are indicated by abbreviations in the specification and drawings, the abbreviations adopted by the IUPAC-IUB Commission on Biochemical Nomenclature or those commonly used in the art are employed. Accordingly, the following abbreviations are used.

| | |
|---|---|
| DNA : | Deoxyribonucleic acid |
| cDNA : | Complementary deoxyribonucleic acid |
| A : | Adenine |
| T : | Thymine |
| G : | Guanine |
| C : | Cytosine |
| RNA : | Ribonucleic acid |
| mRNA : | Messenger ribonucleic acid |
| dATP : | Deoxyadenosine triphosphate |
| dTTP : | Deoxythymidine triphosphate |
| dGTP : | Deoxyguanosine triphosphate |
| dCTP : | Deoxycytidine triphosphate |
| ATP : | Adenosine triphosphate |
| EDTA : | Ethylenediaminetetraacetic acid |

SDS : Sodium dodecyl sulfate
BHA : Benzhydrylamine
Cl-Z : 2-Chloro-benzyloxycarbonyl
Br-Z : 2-Bromo-benzyloxycarbonyl
Bzl : Benzyl
OBzl : Benzyl ester
HOBt : 1-Benzotriazole
DCC : N'N'-Dichlorohexylcarbodiimide
Gly or G : Glycine
Ala or A : Alanine
Val or V : Valine
Leu or L : Leucine
Ile or I : Isoleucine
Ser or S : Serine
Thr or T : Threonine
Cys or C : Cysteine
Met or M : Methionine
Glu or E : Glutamic acid
Asp or D : Aspartic acid
Lys or K : Lysine
Arg or R : Arginine
His or H : Histidine
Phe or F : Phenylalanine
Tyr or Y : Tyrosine
Trp or W : Tryptophan
Pro or P : Proline
Asn or N : Asparagine
Gln or Q : Glutamine

With respect to the human PACAP38 precursor proteins or the mature peptide of the present invention, a portion of the amino acid sequence may be modified, through addition, elimination of amino acid(s) or substitution with other amino acid(s).

The present invention will be described in more detail with the following Reference Examples and Examples. It is understood of course that these Reference Examples and Examples are not intended to limit the scope of the invention.

Transformants E. coli MV1184/pHGP2312 and MV1184/pHGP2306 obtained in Example 2 described below were deposited with the Fermentation Research Institute, the Agency of Industrial Science and Technology, the Ministry of International Trade and Industry, Japan (FRI) under the accession number FERM BP-3054 on August 10, 1990, and under the accession number FERM BP-3033 on July 30, 1990, respectively. These microorganisms were also deposited with the Institute for Fermentation, Osaka, Japan (IFO) under the accession numbers IFO 15068 and IFO 15067, respectively, on July 24, 1990.

Reference Example 1

The amino acid sequence of sheep PACAP38 is believed to be identical to that of human PACAP38. When PACAP38 purified from sheep hypothalami (hereinafter, "Nat. 38p") and synthesized PACAP38 (hereinafter, "Syn.38p") were allowed to act on rat pituicytes in vitro, an increase in adenylate cyclase activity was identified by observing an increase of cAMP observed. The result is shown in Table 1. The minimum effective amount was $10^{-12}$ M, and it was shown that the activity increased with increasing concentration.

Further, similar activity was also observed for synthesized 27-$NH_2$, the amino acids situated in the 132nd to 158th positions of Figure 2 (hereinafter, "Syn.27p-$NH_2$"). In contrast, a similar increase in activity could not be observed for a synthesized porcine vasoactive intestinal polypeptide (hereinafter, "Syn. pVIP).

Table 1

Adenylate Cyclase Stimulating Test in Rat Pituicyte Culture

| | cAMP p mol/ml (M ± SEM) |
|---|---|
| **Experiment 1** | |
| Syn.pVIP $10^{-12}$ M | 1.35 ± 0.05 |
| Syn.pVIP $10^{-11}$ M | 1.40 ± 0.00 |
| Syn.pVIP $10^{-10}$ M | 1.45 ± 0.15 |
| Syn.pVIP $10^{-9}$ M | 1.75 ± 0.05 |
| Syn.pVIP $10^{-8}$ M | 2.55 ± 0.25 |
| Syn.pVIP $10^{-7}$ M | 3.30 ± 0.20 |
| Control (Blank) | 1.55 ± 0.15 |
| **Experiment 2** | |
| Syn.27p-$NH_2$ $10^{-12}$ M | 2.05 ± 0.15 |
| Syn.27p-$NH_2$ $10^{-11}$ M | 2.55 ± 0.15 |
| Syn.27p-$NH_2$ $10^{-10}$ M | 4.00 ± 0.20 |
| Syn.27p-$NH_2$ $10^{-9}$ M | 7.90 ± 0.30 |
| Syn.27p-$NH_2$ $10^{-8}$ M | 9.20 ± 0.00 |
| Syn.27p-$NH_2$ $10^{-7}$ M | 9.20 ± 0.20 |
| Syn.38p $10^{-12}$ M | 2.15 ± 0.05 |
| Syn.38p $10^{-11}$ M | 3.05 ± 0.35 |
| Syn.38p $10^{-10}$ M | 4.60 ± 0.20 |
| Syn.38p $10^{-9}$ M | 6.20 ± 0.10 |
| Syn.38p $10^{-8}$ M | 8.60 ± 0.20 |
| Syn.38p $10^{-7}$ M | 8.70 ± 0.20 |
| Nat.38p $10^{-12}$ M | 1.50 ± 0.10 |

| | |
|---|---|
| Nat.38p $10^{-11}$ M | 1.75 ± 0.05 |
| Nat.38p $10^{-10}$ M | 2.60 ± 0.10 |
| Nat.38p $10^{-9}$ M | 4.60 ± 0.00 |
| Nat.38p $10^{-8}$ M | 8.05 ± 0.35 |
| Control (Blank) | 1.35 ± 0.05 |

Reference Example 2

The materials used in Reference Example 1 were similarly allowed to act on rat pituicytes in vitro. As a result, the releasing activity of prolactin (PRL), ACTH and GH was confirmed therein.

Example 1

Preparation of cDNA Probe Coding for Human PACAP38

The cDNA of cloned human PACAP38 was labelled by the random prime method using $^{32}$P-dCTP for use in screening of the human genomic DNA library.

Example 2

Isolation of Human PACAP Genomic DNA and Determination of Nucleotide Sequence Thereof

E. coli LE392 was infected with a human leukocyte-derived DNA library (Clontech Laboratories, Inc., Catalog No. HL1006d) and plated to cause phage plaques to appear. A portion of plaque DNA was transferred to a nitrocellulose film according to the method of W. Benton and R. Davis [Science 196, 180-182 (1977)] and hybridized with the cDNA probe labeled with $^{32}$P in Example 1. Hybridization was carried out in the absence of formaldehyde at 60°C. Clones positive to hybridization were isolated. Then, a cDNA portion of λHGP23, which was one of the clones described above, was cut out with SalI and recloned into the SalI site of plasmid pUC18 to prepare plasmids pHGP2312 and pHGP2306. By transforming E. coli MV1184 with these plasmids, transformants E. coli MV1184/pHGP2312 (FERM BP-3054) and E. coli MV1184/pHGP2306 (FERM BP-3033) were obtained. The cDNA portions included in these plasmids were 11.1 kbp and 6 kbp, respectively. The simplified restriction enzyme maps thereof are shown in Fig. 1. In the figure, black box (■) shows a mature human PACAP38 code region. The nucleotide sequence of this DNA portion was determined by the method of Sanger [Proc. Natl. Acad. Sci. U.S.A. 74, 5463-5467 (1977)]. This nucleotide sequence is shown in Fig. 2. The region from amino acid Nos. 132 to 169 is the mature peptide portion of human PACAP38.

Referring to Fig. 2, the 7539th to 7649th nucleotides form the first exon, which codes for amino acid residues M(1) to R(37) in the lower row. The amino acid numbers are designated in parentheses. The 9813rd to 9944th nucleotides form the second exon, which codes for amino acid residues P(38) to R(81). The 10420th to 10518th nucleotides form the third exon, which codes for amino acid residues D(82) to G-(114). The 11601st to 11787th nucleotides form the fourth exon, which codes for amino acid residues G-(115) to L(176).

Analysis of the genomic DNA revealed that the precursor of human PACAP38 consisted of 176 amino acid residues.

Example 3

Synthesis of PACAP38 $NH_2$

PACAP38 $NH_2$ was synthesized by using 1.04 g (0.5 mmole) of a commercially available p-methyl BHA resin (Applied Biosystems Inc., California USA) and a peptide synthesizer (Model 430A, Applied Biosystems Inc.).

A starting amino acid, Boc-Lys(Cl-Z), was activated with HOBt/DCC and then condensed to the resin. Thereafter, the Boc group on the resin was treated with 50% trifluoroacetic acid/methylene chloride to deprotect the amino group. To this free amino group, the following protected amino acids activated with HOBt/DCC were reacted in turn according to the amino acid sequence of PACAP38:

Boc-Asn, Boc-Lys(Cl-Z), Boc-Val, Boc-Arg(Tos), Boc-Gln, Boc-Tyr(Br-Z), Boc-Gly, Boc-Leu, Boc-Ala, Boc-Met, Boc-Ser(Bzl), Boc-Asp(OBzl), Boc-Thr(Bzl), Boc-Phe, Boc-Ile and Boc-His(Tos)

After the completion of each reaction, the residual amino groups were acetylated with acetic anhydride to obtain 2.42 g of a protected PACAP38 $NH_2$ resin.

0.51 g of the resulting protected PACAP38 $NH_2$ resin was treated with 5 ml of hydrogen fluoride in the presence of 0.6 g of p-cresol at 0° C for 60 minutes, followed by removal of excess hydrogen fluoride by distillation under reduced pressure. The treated resin was washed twice with 5 ml of ethyl ether, and then extracted with 6 ml of 50% aqueous acetic acid. The insoluble material was removed by filtration and washed with 5 ml of 50% aqueous acetic acid. The filtrate and the washings were combined, and the combined solution was concentrated under reduced pressure to 2 to 3 ml. The concentrated solution was applied to a Sephadex LH-20 column (2 X 90 cm) for elution with 50% acetic acid. The main fractions were collected, and then removed by distillation under reduced pressure. Then, the residue was dissolved in 100 ml of 0.1% aqueous trifluoroacetic acid. The resulting solution was subjected to a YMC-ODS AM120 S-50 resin column (2.6 X 7 cm) and eluted by a linear gradient of 0.1% aqueous trifluoroacetic acid and 50% acetonitrile containing 0.1% trifluoroacetic acid.

The main fractions were combined, followed by lyophilization. Thus, 60 mg of a white powder was obtained. This powder was dissolved in 20 ml of 0.05 M aqueous ammonium acetate. The resulting solution was subjected to a CM-Cellulofine resin column (1 X 6 cm) and eluted by a linear gradient of ammonium acetate from 0.05 M to 1 M. The main fractions were combined. The combined solution was subjected to a YMC-ODS column (2.6 x 7 cm) again and eluted by a linear gradient of from 0% to 40% aqueous acetonitrile containing 0.1% trifluoroacetic acid. The fractions of 28% to 30% acetonitrile were collected, followed by lyophilization. 21.6 mg of a white powder was obtained.

Anal. of amino acids:

Asp 2.90(3), Thr 0.84(1), Ser 2.10(3), Glu 2.21(2), Gly 2.00(2), Ala 3.29(3), Val 3.19(3), Met 1.01(1), Ile 0.87-(1), Leu 2.19(2), Tyr 3.93(4), Phe 0.92(1), Lys 7.18(7), His 0.96(1), Arg 4.19(4)

$(M + H)^+$ by mass spectrography (SIMS): 4530

HPLC elution time: 19.6 minutes

Column conditions

| | |
|---|---|
| Column: | YMC-ODS (AM-301, S-5 120A) |
| Eluent: | A (0.1% aqueous trifluoroacetic acid) |
| | B (acetonitrile containing 0.1% trifluoroacetic acid) |
| | A linear gradient elution from the eluent A to the eluent B for 50 minutes |
| Flow rate: | 1.0 ml/minute |

Example 4

Synthesis of PACAP27 $NH_2$

PACAP27 $NH_2$ was synthesized by using 1.04 g (0.5 mmole) of a commercially available p-methyl BHA resin (Applied Biosystems Inc.) and a peptide synthesizer (Model 430A, Applied Biosystems Inc.).

A starting amino acid, Boc-Leu, was activated with HOBt/DCC and then condensed to the resin. Thereafter, the Boc group on the resin was treated with 50% trifluoroacetic acid/methylene chloride to deprotect the amino group. To this free amino group, the following protected amino acids activated with HOBt/DCC were reacted in turn according to the amino acid sequence of PACAP38 (1-27):

Boc-Val, Boc-Ala, Boc-Leu, Boc-Tyr(Br-Z), Boc-Lys(Cl-Z), Boc-Met, Boc-Gln, Boc-Arg(Tos), Boc-Ser(Bzl), Boc-Asp(OBzl), Boc-Thr(Bzl), Boc-Phe, Boc-Ile and Boc-His(Tos)

After the completion of each reaction, the residual amino groups were acetylated with acetic anhydride to obtain 2.31 g of a protected PACAP27 $NH_2$ resin.

0.50 g of the resulting protected PACAP27 $NH_2$ resin was treated with 5 ml of hydrogen fluoride in the presence of 0.6 g of p-cresol at 0 °C for 60 minutes, followed by removal of excess hydrogen fluoride by distillation under reduced pressure. The treated resin was washed twice with 5 ml of ethyl ether, and then extracted with 6 ml of 50% aqueous acetic acid. The insoluble material was removed by filtration and washed with 5 ml of 50% aqueous acetic acid. The filtrate and the washings were combined, and the combined solution was concentrated under reduced pressure to 2 to 3 ml. The concentrated solution was applied on a Sephadex LH-20 column (2 X 90 cm) for elution with 50% acetic acid. The main fractions were collected, followed by removal by distillation under reduced pressure. 129 mg of a white powder was obtained. This powder was dissolved in 5 ml of 0.1% aqueous trifluoroacetic acid. The resulting solution was subjected to a TSK-GEL (ODS-120T) column (21.5 x 300 mm) and eluted with 27% acetonitrile containing 0.1% aqueous trifluoroacetic acid.

The main fractions were collected, followed by lyophilization. 17.2 mg of a white powder was obtained.
Anal. for amino acids:
Asp 1.99(2), Thr 0.98(1), Ser 2.76(3), Glu 1.25(1), Gly 1.05(1), Ala 3.00(3), Val 1.56(2), Met 0.78(1), Ile 0.72-(1), Leu 1.88(2), Tyr 2.22(3), Phe 0.75(1), Lys 2.73(3), His 1.51(1), Arg 1.94(2)
$(M + H)^+$ by mass spectrography (SIMS): 3145
HPLC elution time: 21.2 minutes

Column conditions

| | |
|---|---|
| Column: | YMC-ODS (AM-301, S-5 120A) |
| Eluent: | A (0.1% aqueous trifluoroacetic acid) |
| | B (acetonitrile containing 0.1% trifluoroacetic acid) |
| | A linear gradient elution from the eluent A to the eluent B for 50 minutes |
| Flow rate: | 1.0 ml/minute |

Example 5

Using male Wistar rats having a body weight of 350 g under nembutal anesthesia, the hypotensive activity was measured. The value shows the decrease from normal value. The results are shown in Table 2.

Table 2
Hypotensive activity of PACAP

| | Dosage (n mole/kg) | | |
|---|---|---|---|
| Compound | 0.3 | 1.0 | 3.0 |
| PACAP38 $NH_2$ | 3.2±1.9(n=6) | 17.4±2.4(n=6) | 29.8±3.6(n=6) |
| PACAP27 $NH_2$ | 14.5±3.1(n=5) | 51.9±9.6(n=5) | 4.1(n=1) |

Unit: mm Hg

## Claims

1. An isolated genomic DNA of human PACAP38.

2. A genomic DNA of human PACAP38 according to claim 1, containing a nucleotide sequence coding for human PACAP38.

3. A genomic DNA of human PACAP38 according to claim 1, having a nucleotide sequence as shown in Figure 2.

4. A genomic DNA of human PACAP38 according to claim 1, having four exons coding for a precursor protein of PACAP38, said precursor consisting of 176 amino acid residues as shown in Figure 2.

**5.** A genomic DNA of human PACAP38 according to claim 1, having a 5'-region wherein said 5'-region has a promoter region.

**6.** A genomic DNA of human PACAP38 according to claim 1, having a promoter region wherein said promoter region has a recognition sequence.

**7.** A genomic DNA of human PACAP38 according to claim 6, in which said recognition sequence is selected from a group consisting of cyclic AMP response element (CRE) and 12-O-tetradecanoylphorbol-13 acetate (TPA).

**8.** An isolated precursor protein of human PACAP38.

**9.** A precursor protein of human PACAP38 according to claim 8 which contains an amino acid sequence consisting of up to 176 amino acid residues as shown sequentially in Figure 2.

**10.** A transformant containing a genomic DNA of human PACAP38.

**11.** A transformant containing a DNA sequence coding for human PACAP38.

**12.** A transformant according to claim 11 which comprises Escherichia coli MV1184/pHGP2312(FERM BP-3054).

**13.** A transformant according to claim 11 which comprises Escherichia coli MV1184/pHGP2306(FERM BP-3033).

**14.** A transformant in accordance with claims 10 or 11, wherein said transformant was produced by an animal cell host.

**15.** A method for preparing a mature PACAP38 protein comprising:
1) cultivating a transformant containing a genomic DNA of human PACAP38 or containing a DNA sequence coding for human PACAP38,
2) accumulating the protein in a culture product to form an accumulated product, and,
3) collecting the resulting accumulated protein.

FIG. 1

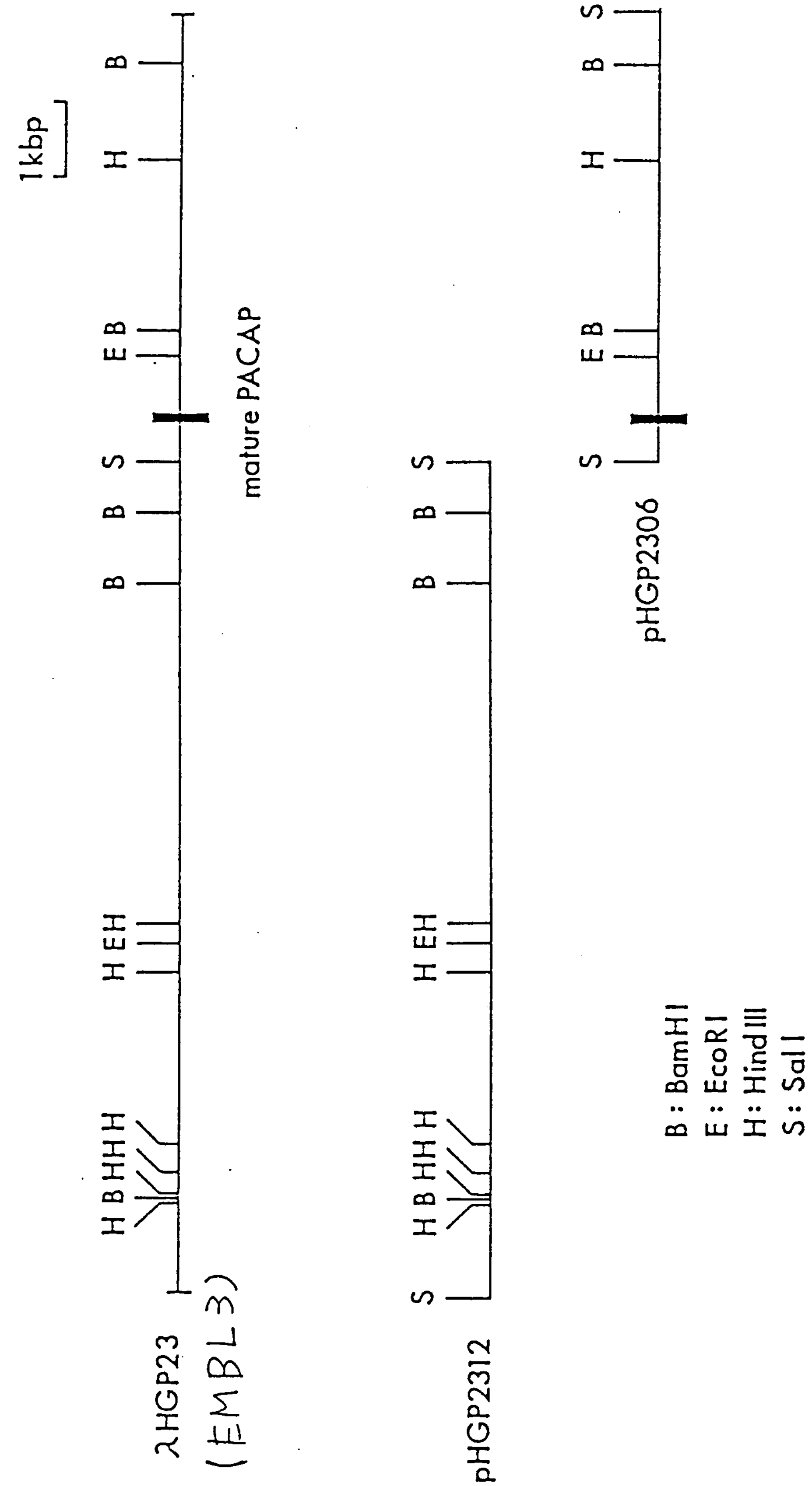
1kbp
mature PACAP
λHGP23
(EMBL3)
pHGP2312
pHGP2306
B : BamHI
E : EcoRI
H: HindIII
S: SalI

FIG. 2—1

```
   1  GATCACGAGGTCACGAGATCGAGACCATCCTGGCCAACATGGTGAAACCCCATCTCTACT
  61  AAAAATACAAAAAATAGCTGGGCATGGTGGCCCATGCCTGTAGTCCCAGCTACTCGGGAG
 121  GCTGAGGCAGGAGAATTGCTTAAACCCGGGAAGCGGAGGTTGCAGTGAGCCAAGATCGCA
 181  CCACTGCCTCCCAGCCTGGTGATGGAGTGAGACTCCATCTCAAAAAAAAAAAAAAAAAAT
 241  TCCTAGAGAAAATAAATATGCCAGTATAACATATTATAGTCATTAAGACTGTCTGGAGTC
 301  ATTGAGACTTGAATCTGAAGTTCAGCATTACAATGTAGCAGCTGTGTAACTTTGGATAAG
 361  GTACCTGAGCTCTTTTAGTCCCGATTTCTTGTCTGTAAAATGGAGGTAATAACAGTGCCT
 421  ACAAAGAAGTTTGTTGTGAGGGAAAGGAAATAAGTAGTCAAGCACTTAGCCCAGGAAGTG
 481  TTCATTAAACAGTTGTTGCTGTTGCTGTTATTCACTGGTGAATAACAAAACCATACAGTC
 541  CCTTTGGAAGGAAGGATTTAAAATAATTTAAACAAATAATCACTAAAAATTTCAACCAGT
 601  ACATTTATGACAAATGTAATAGTATTCCAAGCAGATGATAGTTTTTAAAAATTTATGCCT
 661  GTGTATATTTGGGTAGAGACAAAGGATTATTTAAAAAGTATTTTCAGGTTGGGCACACTG
 721  GCTCATCCTTGCAACCCCTGCACTTTGGGAGGCTTGAGTCCAGGAGTTTGTGACCAGCCT
 781  GGGCAACACAGGGAGACCTTGTCTCTGCAAAGTAATAATAATAACAATAATAATAAATAA
 841  AATTAGCCAGGCATGGTGGTGGCATGTGCCTGTAGTCCCAGGTATTCAAGAGGTTGAGGC
 901  AGGAGTCTCACTTAAGCCCAAGAGTTTGAGGTTGCTGTGAGCTATGAATGCACTACTGCA
 961  CTCCAGCCTGGGTGACCGAGGAAGACTCAGTAAAAACAAAACAAAACAAACAAACAAACA
1021  AAAACTGCAAAGCCGTGATTACCATACAGTGCTAGTAATAATGATAATAAAAACAAAGGC
1081  TCCAAAATTATTACATGTAAACCTATATTCACAGGTAGATAATGCCAAGCCTGAGCCCAA
1141  AGGGAAGAGGGATGCTAGGGGCTCACAGAGGAAGACCTTCTTTTGATTTTACACACAAAA
1201  ACCTTGTTTTATTTTGCATATAGATTCCCCTTCCAACATTTTCTGAAGTGGTCTCAAAAG
1261  CTTATTTAGGATATTGGTTTTCCTGGATCCTGTCCAAGCTTTTCCTTCTGCATTTAAGCC
1321  TTATGTCAGCAGAGGATTTAGACTAAGTAGAGAGAAGACTTTCTTCTCCTTGGCTTTTTA
1381  GAGGAGGTGCTCTGGAATTTGGAAGATGCTACACAGTGAAGTCTGGGATATACATTTTTG
1441  GTACCCAGTAAACTCATGTTCAGAGAATAAGGCCTTAGTAGAGCCATATATGTGAGATAT
1501  TTGGAATCATCCAAACCTGTAGCAAAGGTTAGATCCTGGTTTTTGTTTCTCAGATGTCTG
1561  CTTTATATCTCAAAACAACAAGCAAAATTTCTTGGCTCTGTGAATATGCAATTCTGTTCT
1621  TAGATTAGAGAGAAGCTTTACTCATGTGGTTTGGAAGGCTTCCTTTCCTAGTTGTTTTCA
1681  GTGTGTGAGAAGCACTACATTTTGAAGGTCAGAGAAGTCATGACACATTATAGGTAAGCT
```

FIG. 2—2

```
1741  CATCAGCTTCTTACTTCACAGTGAGTTCTGAAAGGCATGATGCATGCAGTCCAGTAAGTG
1801  ATGGTCATGATGTTCTGGTTCAGAACATTTGGGTTTCCCTACAGGTGTAATCGGTATGAA
1861  GTGAGTCATTAGTCATTGCATTTTCTGGAAAAGTCGGTAGAGAAAAGTTTAAGTGAAATG
1921  TAATACAGTGTTATAATTCACTTTTGTCACTCACAGGAGAGGATGATGTTTTGCATCAGG
1981  CTTGTTTACTGAAAAAGCTTATTATAGCCTGGTTCTTATGCTAAGTACTGGCTAAAAAAG
2041  AATAGAATGTGCCAGGCACGGTGGCTCACGCCTGTAATCCCATTACTTTGAAAGGCTGAG
2101  GCAGGTGGATCACAAGGTCAGGAGTTCGAGACCAGCCTGGCGAACACGGTGAAACTCTGT
2161  CTGTACTAAAAATACAAAAATTAGCAGGGTGTGGTGGTGGGTGCCTGTAGTCCCAGCTGC
2221  TTGGGAGGCTGAGACAGGAGAATCACTTGAACCTGGGAGGCGGAGGTGGCAGTGAGCCGA
2281  GATTGTGCCACTGCACTCCAGCCTGGGGGACAGAGCGAGACTTCATCTCAAAAAAAAAAA
2341  AAAAAAATGCACAACTTTTCCAATCTTGATTTAGATTATTTATACTAGGAATGTGTGAGG
2401  ATGCCTTGAACAAACATGTCCTTTTATATGGTTAAGAAAATCAAATGTTGTAGGATATAG
2461  AGATAGTGGTAGTAAAAGGTAATGGTGTAGAGATATGTACCTAAGGAAAGAGAATGTCAT
2521  GGAGAAACCCTGGGTACTATGGGTGACTGAGCCAAAAAGAAAGTAGTGGAAATCTTATCT
2581  AAGTGACCAGAAGCCGCACTTCACTGGGCTGCTTAAAGGCAAAAATACTTTTAGCTCACC
2641  ACTGATTTGCAATATGGGGATGGAGGGGAGCAGTGTTTAAAAGATGCTGAAGATTCCCAT
2701  GCAATATAGGGAAAGCCAATTTCCCAAGTGGTGATGGTCCAAAGGCAGGAACCTGGCACA
2761  GACACAACAGTACAAACACTATAGTATTTGCTAATGTTGTGAAGCCATCTGCAATTCAAA
2821  CTCCCAGTATATATACTAGACATATTCCTCCTGTTTGAATACAAAAACCCACTTCCTCAA
2881  AGGCTAGAGTTCTTTAAATTGAATGTTAATTCAAGGTTCAAGGATTAACCCTTCAACAAA
2941  GGCGGATGTGTTAGCCACCAGGAAAAACAATTCGGGGAAGGGTTAGTTTGACTTTTAGCT
3001  ATTATTTATCATTTCTTACCCAAACTTGTTTTCACATCTGAAGGACCAACAGGATAAAAG
3061  TTGATACATTAGGGACTTGAAGTTCAGAGTATTATTAAATCATTTCCAACAAATATATAT
3121  AAACAGCGTCTTCCGGGCAGGTCAGGGCTTAGCTCAAGTCACTTTCAGTTGCTGTGCCTC
3181  AGGGAGGATGCTGGTTAGACCTCCCACTGAAAGATTTCCATTGTTCTTCTAACTTTTCTA
3241  GCCAAACATGATTCCAGTTAATGTAACAATCTCATAGCCTGGAAAGAAACTGCCAGCCTG
3301  GGAAATCTACTTTTCTGGCCTGGGAAGTATTCTGGTGAGCACTGAGGGAAGGGAGTAGGG
3361  GTGTTGGAAAGAAGACTTGAAATTCCTTTGTGTTATCTGTAAATAGAACGTTCTAACTCT
3421  TTGGTCTCTTCTTCCTCCTCTCCCCCAACCCCCTCTTTCATCATTTTCAATTATATATAG
```

FIG. 2—3

```
3481  GAGGTTGGAAAGTTTCTCTTGAGCTCTTAACCCCAGTCACCTAAATACCCTTTGTGAGGG
3541  AAACTGGGTAAGAACAATTAAAGTGGAAGGCTCTCCTACCCTGGTCTTGCTCTTCCCCAA
3601  TTCTCCTCTAGCTCCTCCTCCCTTTATCTCTCTCTCTTCTCATAAAAGTGCTTTAGTTGA
3661  GGCTTCCTAGGATTCACCCTCCAGCTCCTATCTGCACTTGAAGCCAGGCTGGGGTCTGCA
3721  CTTGCAATTAGTATGTCTGTTGGACTGGGCCACGGTATCCCACCTGGCCACTGCCGCATG
3781  CCTCCTCAGTGCATGCCGGGGCTCCTGGTCTCTCTAGCCTGGGGCTTTGGGCTGACAAGT
3841  CCCCTCTTCCTTGCAGCTCCCTCAAAGTCCCAGACACAAAGGCCTCCAGGATGCTCTGTT
3901  AATGCTTGACTGGAGCCTTCCAAGATTAGAATCAAAGGGGCATTTGGGGGTAGTTTTGGT
3961  CTTTGAGACTTCAGTCATCCCATATTCCCTCTACCCAATAGAAAGCAGAAGGGGCCTATA
4021  CTCTCATCTAGCAGCTTCTAGTTCCTCCTATTTATTGGCCTTTTCCCTTGGCCCAGGGCC
4081  AAGGCCAGATTTTCATGAATAGGAAAGCTCTCCTGCAGAGAGATGTCAAACATGCCCAGC
4141  TAGACAATGGCCATGAGCAACAAAAGATCTGTGGGTGATCCTGTAGGAGTTTGATTCCCC
4201  CAGGCTGCTGTGGGCAGGGCTGTGTGGGTCTTTAGATTGTGTTGAGCAATTGGTGGGTCT
4261  AGGAAGCTTGATTTTCTGGAGTTCAGTGACCTTGAATCTCAAGTTCTCTGTTTAGTCTTT
4321  CACTCTCGTGAATGGGTTCAGGTCTGAAGGCCTGTAATTTTTGGGTGCTGAGCCCCGAGT
4381  TCTGAGCTGAGAGTATCTAAGCTGAGAAGAGCACGGGGTCACAGCCTTGGTAAGTCAGAG
4441  GCACAGTTCAGCCTCTGTTGGCCCTTGGAGCCAGCAGTTAGTTGTCCTCCCCAGATAGTT
4501  AATCGTGTTTGTGGTTTTCCCCCTTTAATGGGCCGTGAAGTCAGCAAACTCCCCACATGG
4561  TGGCTCCCTTTACTAAAGTTGAGTAGTGAATTGTACAAGGAGTCTTGGAAATTTTCAAAT
4621  ATTTCTCCAGATTGAACTCAACTCAGAATTCGTGTGGGAGGAAAGAGTAAGGATTTTAAT
4681  GGGGTTCACCTTTGACGTGAAGCAAGGCGGAAGACAGGAAAGCCACAGTGGGGAATAGCT
4741  TTGGGCGCTTTAGTAAGAAAGACATCTCTGCTTGATTATCTGGTAGTGTTCACCGCAGGC
4801  TCTTTGTGTGCGAGCTTGCTGCAGAGGCAGAGCTGAACACGGAAAACACGCATGTAAACA
4861  GTCCAACATAAATCAGTGAGCATATGTATCAGAGAAAAAGAGACATATTCCCATGTAGAT
4921  GTGGTTTGAAGCTTTATTGAAGGCAGACAATCTGAAGCACGGCCAGGAATAAACTAAGAG
4981  GAAGCAGACAGTTTTCGGTCATTCATGGCCAGGAATAAACAAAACTTAGTTTTTTTTTTA
5041  AGAAGGAGGAAGTATTAAATCTCAAATAAGAGCAGGAACAGCATTTAGAAGGAGAAATAT
5101  AATATCTTGGAAAAAGCAAGCAGAACTAATATAGCTTATTTAAATGTGAGATCCAAATCG
5161  TAGTAACAGGAAACCCTCCCACTAAACTGGAATTTCCCCTAATTTTTGTGTAAGATCCAA
```

FIG. 2—4

```
5221  ATAATTAAAATGCACTCTAATGGTTATTGATGGCTCTATTTTCTTTCTTTCTTTCTTCTT
5281  CTTCTTTTTTTTTTTTTTAAAGAAATAGACCTGAGTTCTCTTACTGGAGTAGAAATATAT
5341  GAACCTTCTTCAATTACCCAGGAAATTGGAAGCCTCTGGGTGGATATGGTCTTCCCTTAT
5401  TGCTTTCCTCTTCCCACATCATTTTCAGTTAAAAAAATTAACTGTTTCCAGCAGAGGGAT
5461  TCCTGTTAGAAACCTTCATCAGGTGAACTTGTACTGGGAACCCTCATGCTTTCCCAGTCT
5521  GTCTGTGTCTCCCAAACAGAGCTGAAGTTGTAAACAAAGTGGAAAAACATATTTCTCACC
5581  CCAAAATTCTTAAAATTTCACTTCTTGTGGAAAACACAATTTCACAACATCAATTTTTAA
5641  AATCTGTAAGAGCCACAGAAGGTGTGAAAGTAGCCAAACAGCCGGTCTAGAAATCCAAAA
5701  GCCAGGACTAACGGGGGACAGAATGCTTTTTCCTCAAATCCAGGCAGGGATGGGGAGCAT
5761  TCTCAGCATTAGGGCATTTATGGACGCTACAAGGGGAAAGGATGTATCTGAACGGTGGGG
5821  GTGATTTAGCGATGAATCGCCACGTTAATAGCACTACTGCCAAATCTTCAAATTTAGAGG
5881  CTCTGGTGAAAAATTAAACCGGTGGCAATTTTCAACGTTTGTAGCATCTGTTACCCTACA
5941  CTTCAGCACCCGGAGTCTGGACAGCTCCGCAGGCCGCGCTCCGGAGGCAGCATGAGCTCT
6001  CATCAATCTACTCATAGCCCTACTGTCAACGGCAGCCAGACTCAGGAGAGATTTACTGAA
6061  AATCCTCCAAGACTTCCCTTTAAAAACAAAACGACTTCCACATTTAATGGTCTATCTGAA
6121  AGAACATACGCAAGAAATTAGGAGATCTAAATTAAATTTATTAATAGGAGAGCTTGATGA
6181  TGCTTAATTCCAGAGACCAGAGCTCCGATTGGTGAGGCTTGATGAAAAAGTAAAGAGAAA
6241  TCGTAATTGTATAGTTAAAAACATAACTTTTGTCATCCTCAAAATTCTAAAAATTCTTTA
6301  CCTGTCCTTGGGAAATGGGTGAAATTGAAAACCATCAAAACAATTGGACTTCTTAAAAAT
6361  TGGATTGTATGAGTGAAAGGTGTTTATGAGAAGTCGATGACTCCGGATCTTATCATCCAA
6421  GAGGACAGCACAGAATAGTTAATATGTTCCTTGAGGGACTAGGATGCTGACGTCTTTTTC
6481  TGATACCCGATCATTACGTGACTGAGAAAAAAAAAAAGGAAGTCATTTCATGAATAAAAA
6541  TCGGAGCGCAACAGTGCAACAAAATATTCTGTACTTAAAGGCAACAGGCAGGCAGATGTT
6601  GACAAAGAGGGCTCTCCAAAAACCATGTTCGGATAGATTTTTGCGAACTGCACAGATAAA
6661  TAGGAGCAGAAGGCCGGTCACCTCTGTAACCAGCGGTAGCAGCAGCAGAAGCCGCAGCTT
6721  CAGAGGCAGCCGGAGAGACCTCGGAGCAGAGAAGGCGCCGCCGACCCTCGCGGCTGCCTG
6781  GCCCGCGGCTCCTACAAAGGCGGGCTAGCCGCCCGCCCTCTCCCTTGCCTTCCTCCCCTT
6841  CTTTTCTGACTTTCCCTCTTTCCCTTAATCGCCTGCTTCTTCCTCCGGGTGGACTTACGG
6901  CCACCTTGCTCCTCCGCGCTTCACCTCATCGCCCCCTCTTTCTTTCTTCTGCCTCTCTCT
```

# FIG. 2—5

```
6961  CTGCGCCCCCTTCTCTCCGTGTCACGCTCCCTCCTGGTTCTGCGCGTCTACAAACTTTTG
7021  AGCAGAACACGAGCCTCGGCAAACGAGTCCCGCAGCTCCTCCTGCTGCTCCCGCTGGTTC
7081  CTGCGGCTTCTGCTCAGACACCAACGCCAGACGGCGATGCCTCTCGGGTGGTGACTCCAG
7141  CGCAGGAACTTGAAGAAGCGCTTTGCCCGCCGTCCTACCTGGCAGCTCTCCTGGCAGCGG
7201  GAGGAGTTGAAGGGTAAGGGAGGGAAAATCTTACCAAAGCGACCGGCTCACTCGACTGCT
7261  GATTCTTTCGCTTGGCGTCGCGTCAGGGGAGTTAGCTTTCCTTCAGCCGGGTCTGGCTAG
7321  TTATTGGGCGCCGGGTAGATGCATATATATATATTTTTTTCTAACTATAGCAAGCAAGAA
7381  GTGGCAGGGCGCGCACCGGCTGTCGCCAAGTGCTGTTCAACTCAGGGAGCCGGGGCTTCG
7441  CTCCGTCCCTCCCCCGGCTTCCAGAGCTTTTTGGGGTTGGAGGGTGGGAGGCCAGGGGCG
7501  TTCTCACAGCTGTGTGTCCTCTTTCCCATCCTGCGCAG

7539                                              AATGACCATGTGTAGCGGAGCG
   1                                                M  T  M  C  S  G  A
7561  AGGCTGGCCCTGCTGGTCTATGGGATAATCATGCACAGCAGCGTCTACAGCTCACCTGCC
   8   R  L  A  L  L  V  Y  G  I  I  M  H  S  S  V  Y  S  S  P  A
7621  GCCGCCGGACTCCGGTTCCCCGGGATCAG
  28   A  A  G  L  R  F  P  G  I  R

7650                              GTAGGTGCTGGCTGCCTGGCCCAAGCAGGAG
7681  CTGGGGCTCCCCAGGCACAGACGCTTCCTCACGGTCTCCTTCCTGCAGTCCTTTGGGTCC
7741  AGACTACTAGCATCGCCCTCTGCGCCCCCGGTGCGCCTCCGCCAGCCTCGGCTGGACAGC
7801  GGGTCCCCATTCTAGCCGAGGGTCTGGCAGGCTCCGCGACTGCTCGGACGCCTCCCCCAG
7861  CCCTAGGCAGCTCAGGGTCCCGGGTAGAGCCAGTGAGCTTCTGGCCGCTGGAGAACCCCC
7921  CCTCCCCCAACCCGGCCCACAGGATGGGGGCAGGGCACGGCCCCTAGCTTGGTTTCTTTT
7981  ACCTATTCTTGGGACGAGTTAGGAGAACTTCAGCTCTGGAGCCTGGCCGGGGGTTGAGCG
8041  TGAAGCTCCCTCGGACTTTGCTTTGTTACTGCTTGTTCTGGACTATCCGGGTGGGGTCTC
8101  TCTCTCTCCTCCACCCTTTCTTTTCATTTCATTCCAATTCTTTCCCCTGAAGAGCTTTCT
8161  TTCAAGTGATCCGTGTTCCAACTGCATTTTGAATCCCAGGCTGTCTTGGGGGGCGTGCGG
8221  TGGGGAGGGTGTTGGCCCGGTGTGATTGAGGAAAAGCGACTTAAGAGAGGGAAGAACAAG
```

FIG. 2—6

```
8281  GACGAGACTGCGAAGGAGGGGAAAAACAGGCGCAAAGGAGGAGGAAGGGAAAGCCAGCA
8341  GGCAGGCAGGACCGGGAGAGCAGCCCTGCCTGGCCCGGGATGGAGGAACCTTGGCTTTTT
8401  TCTTAACCCCGGGTTTCTAACCCGCAGGCGCGGCCCAGGTTCCCGGAGGCAGCCCCAGAG
8461  TCGCGGGCCGATGTGCCAGGCTGTGGATGAGCCCCGGGTAGGGGAGGGTTCGTACCAGCG
8521  GCGCCTGGGGCAGCGAGGAGCGCGCGTTCTGCCTGCGAAGCTGCCTTCTCCGAGCCCCGC
8581  CCAGGAACATTAGCTCTGGGGGGCCGCTGATCATTGATTTGGACGGAGAGATGGGTTCTG
8641  GGTTCTGTATTAGGATTCCAGCATCTGGGCTCGAGGCAGGGCAATATCCAGAAAGACCCC
8701  AGGGTTCGGGGTACCCGGGCCAGGGCTGAGGCGCATCGCCGAGCAAAGGCTGGGTGCGAG
8761  GCGTGCGGAATGATGCGCTTGCCTTGCCCGGGCCTCTCCAAGGATGGAGAAAAGGCGAGT
8821  GAAGTAGCGAAGTACGACTCCAACCCCGCCCAGAGAGTGCTACTAGCGCTGGCTGCACGC
8881  CAAGTCTCTCCAGGGGTCCAAAGCGAGAGGGATTTGTTTTAACCCATCTCTACCCGTCCT
8941  GTGTCAAGAACGGAGGCTGTAGAGGGCGACTGCGAAGTCGCCAGGCACTCGCTGGATCTC
9001  GGTCCCCCTCCTCGTGCTCTGGGGTTGAGATGGGGCACCGCCATCGATAACAGATCAGCG
9061  CGAACTATTCGTTTAGTGGCCTTAAAACACCCTGGTTTCACCCTCAGCTATTTTCAAGTT
9121  CCCGTGTGCCTGGCACTTTCTCCGTGCGAGAAGCACCGGAGGGTGCGGACGCGCCACAGT
9181  CTGAGCCGCCGCCGAACTGGCTAAGTTTAGGGGCATTTATTATTCATGTTCCTGCCAGAT
9241  CCTCGCCTGCCCAAAATAGAAACCGAGGTTCTCCGTGACCTACATCTGCTCGGGGAAGGG
9301  CTCCCCTGGGCTCGGAGGCTGGGGTGGGGGTGGCTGAGGAGTTGGCCCCCGCACGCCCCA
9361  CGCATCCTCTCCTTTGCTTTCTGGGCCTCCCCATTCGGGTCTTCGCGTGGGTCAGCGCCC
9421  GGTCTCCCAGGGCCTTTCTCGTCCCCGCCCGTTGCTGCTTTGGGGAGGCTCGGGAGCCAG
9481  GCGGGGAGGGGGGCGGTCCTTTTCCGTAGACAGGTGTGCGCGATCGGCGGAGACGCCTCG
9541  GTTTCCCAGCGCTTGTTGAGGCCGTGGCCCGCAGGACGACCCTTTACCCGCGAAGGGGGG
9601  GTGGGCGGACCGCCCGGCGGGGTAGGAGTGGTTGGGTGTCGTTGCCTCCTCCTTACCTC
9661  TGCTCCCACCCCCAGTCCTGGGAGAAGAGACAATTCTCAGCGGAGGACTTTTATCACCTG
9721  TGAAAATCCGCGCGAGCCCCTTACTTTGGATCCTCGCCGAGCTGGGGAGGAACTTGCACT
9781  GACCACACCTTCTGTCCCCGGCCACCCCGCAG

9813                                 GCCAGAGGAAGAGGCGTACGGCGAGGAC
  38                                   P  E  E  E  A  Y  G  E  D
```

FIG. 2—7

```
 9841  GGAAACCCGCTGCCAGACTTCGGTGGCTCGGAGCCGCCGGGCGCAGGAGCCCCGCCTCC
   47   G  N  P  L  P  D  F  G  G  S  E  P  P  G  A  G  S  P  A  S
 9901  GCGCCGCGCGCCGCCGCCGCCTGGTACCGCCCGGCCGGGAGAAG
   67   A  P  R  A  A  A  A  W  Y  R  P  A  G  R  R

 9945                                              GTGAGATTCGCGCGGC
 9961  CTCGCGCACACCCGCGGCTGGGAGCTCGGGACTGCGGTGACGGGAGGGGCAGTGTGGTGA
10021  CCCACCCAGGATTTTTTTTTTTTTTCCCGTGAAAGTCCTCAAGCCTGTCCTCTCCCTGGC
10081  CCGATCCTATTGCAGCGACAGAAAATCAGCAGCGGGCGGGTCTGTGTGGACCTGAGGGCC
10141  GCGTGGGGACCGAGGGGGGCTGTGGCCCAAAGAGTGGCAGTGAGTGGCGTCAAGGAACCC
10201  ACACTCCGCATCTGCCACTCCTAGAGCCGGGACTAGCTCCCGATCCTAGCAGTTGCTCTC
10261  GAGATCATCCCGGGAGTTATTGGCGAGTTCTGGGCCTCTGGAGGTTTCCCTGTCAGCCTC
10321  CCCGGCCGCCGAGGGGGCGCGCGCCCAACAAGGGGGTCTCTAGCGGCCACCTGGGGACAG
10381  AAACAGTGACCCTGGGCGCGCACTTTGCCTCCCCGTTAG

10420                                           AGATGTCGCCCACGGGATCCT
   82                                                D  V  A  H  G  I  L
10441  TAACGAGGCCTACCGCAAAGTGCTGGACCAGCTGTCCGCCGGGAAGCACCTGCAGTCGCT
   89    N  E  A  Y  R  K  V  L  D  Q  L  S  A  G  K  H  L  Q  S  L
10501  CGTGGCCCGGGGCGTGGG
  109    V  A  R  G  V  G

10519                   GTAAGAGTTTGTGGAAGGATTAACCTGCGCGCGCCGGGGTGG
10561  GTGCCTGTGCGGGGCGCGCGGGGCGGCGGCGGTGGGTGCCCGTGGGGGCCAGGGTGAGT
10621  CTGCGCCCCTGGGTCTGGGGTGGGCATCCGCCACGGGTCGCAGTTGGAGATTTTGAAGTG
10681  GCACTTTAAATTTGCCCAGAGAGCTCTGGAAGAGGCAAAAAGGGAACGCGAGCCAGGGAG
10741  TTTGATCCGTTTTGAATGAAAAGAAAGAGAAACCAAACCAAACCTCTCAGTCATCCAAAA
10801  CCTTCAGGCTTCCAGGGAGGTTTTGCTATAATTTTCTCTAAGCATGACTGTTTCTGGGGG
10861  AGGGGAAAGGGGTGGTTGTATTTACTGAAAATTCAAATCGAAATAATAAATGGCCAAATG
```

# FIG. 2—8

```
10921  TGGACACTTATGGACCCAAACAGTTTTGCTCACGCCAGAGAAACTGAGAGCACAGGGCTT
10981  GCGTGAAGCCTATCTCGGCAGAAGGCAACATTCTAATAAAGCCCGTGGGAAAACAGATTA
11041  CATTTTCGCCATGAATAAGTCATGCAGTGAAAAATATTGCCTACAGCCTGTCGACTTATA
11101  TTATTATCACGTTTTTCAACTCGGCGTGAGGAGGGAGAGGAGTGTTCATATTTGACTAGG
11161  AATTGCAGGATCGATGCAAACTCCAGGGCAGCAGCCAGACTGGCATATGTAGGGCTCTCC
11221  GGTTACTTTCTCTGTATGTCGCGGGTGAGAGGAACAGCGAGGACAATTTAGCGCAAACAC
11281  ACGAAGGGTCGGATCTCAAGGGGGCAGCGCTGGGAGAAAGGTTAGGCTTGAAGCGCGCGT
11341  CGCCTGCCCGGATCTTATCCCGGCCCCCTCCGCAGGGTTTGGTGCCAGGAGATCCTGCG
11401  TGGGGAGGGGGGCATCGAGGGGCTGCCGTCTCGGCCCTCCCCACGGCTGCTTCCAGGCAG
11461  AGGCGGGCGACGCGGTGGGCAGTGCGAGCCCCGGCCCTCCCCGAAGGCTCCCGCGTGGG
11521  GTGGGGCCCGCCTGCTCCCCGCGGCGATTGAACCTGTGTCTCCCGCCCCGCCACCCTCTT
11581  CCCGACCCCTTTGCTTGCAG

11601                      TGGGAGCCTCGGCGGCGGCGCGGGGGACGACGCGGAGCCG
  115                        G  S  L  G  G  G  A  G  D  D  A  E  P
11641  CTCTCCAAGCGCCACTCGGACGGGATCTTCACGGACAGCTACAGCCGCTACCGGAAACAA
  128   L  S  K  R  H  S  D  G  I  F  T  D  S  Y  S  R  Y  R  K  Q
11701  ATGGCTGTCAAGAAATACTTGGCGGCCGTCCTAGGGAAGAGGTATAAACAAAGGGTTAAA
  148   M  A  V  K  K  Y  L  A  A  V  L  G  K  R  Y  K  Q  R  V  K
11761  AACAAAGGACGCCGAATAGCTTATTTGTAGCGATGGGTTACCAGCTACCCTGTGTATACA
  168   N  K  G  R  R  I  A  Y  L  *
11821  GCCCTGACGCAATGAAAAGTCGTTTTCCAAACTGACTCAACAGTCATCGCTCGTGTGTTC

11881  TATCCAAACATGTATTTATGTAATGAAGTAAAGCCATTAAATGAATATTTTGATAATAAT

11941  ATTGTTTTTCTTTCTACAAAGCACTAGAGAATGCACAGATATACTTTGTGGACCAATTAT

12001  TGATATATATTATAAATATATATAAAGAATATATATATATATATATATATAAAGTATAGA
```

FIG. 2—9

12061 GAGAAGTTCATACAAAGCGTGCACAAGGATTGAAAATTCGCCCGAGCTGTTTATGTTTTT

12121 ATAAAAATAAATAGAAAAGTAGACAATCATTGTTTTGAATATTACTCCTATTTTTGTAAA

12181 CTGGAATTAAAAGGATAGTATTTTTATCCATGACAGGCCTGAAGATATTACTACTTACCA

12241 TTTGCTACTGTACATAAACAATGATGCCCTGCTCCAGGGAGATTTTGAGGTAAAGATATG

12301 GAGAATTGCTGAAGGGCATTCTTTCCCAGTGAGTCTCTGGGGCAGGCTGCTTCAATCCCA

12361 GCCTAACTCAACTGGGCTCTGTCCCCCTGGTTGGGTGGCAATTCCAATATTTCTGCTTTC

12421 TTTGATTCTCCTTTTATGTGTAGTTGTCTCTCTTCAGACTCTCAGCCCAGAAGAAAATTC

12481 TCCTGATAAAACAACAGCTCGATCCAAATTGTGCTTCTCCCCAGAATTCACGCCTCTCCC

12541 TAGGAGAAGAGTTGAGGAACTGTACAGAAAAGGGCGGCTTCGTTAGACCGCTCTCTTTTC

12601 TGTACTTCCTGAGTGGCCAGGGAATCTAATATCCCCAAATTAGGGCAATTGGAACAAAGT

12661 GAAGGACATAGAGGTATATTGGAAGAGGCAGAGCCTGAGGTGGTAGGAGGACGACCCTGG

12721 AAATGGACTGGTTTGAGATTGCCCCAGGTCTGGGAAGCTGAGGGCAAATCCAGTCCCAGT

12781 GGTCCTGACTTTGGGCGCTGGGTATTGGAAATGGATGCAAAGTACAATGTGTTTTTCTCC

12841 AGTGCTGTCCATGCTTCTCATCTTGTGAAATGGCCAGGATCCTCTCCTTTGAAACCTGCT

12901 CTGTAGGAGCTACCCTTTTCCTTTGTGGTTTTATGGAGACCTCTCCTTCCTACCCTCCTG

FIG. 2—10

12961 CACTGTTTAAGTACTGTTTACCATTTTTCATTCACTTCTCTTAAACTTGTGAATGCTTCT

13021 CACTTTTTTTTTTGTTTGATGCAGGCACTTATTGTAAATTTTAGAAACCCCTCTGTAGCC

13081 ACTAGTAAGTAATTATGCACTAAATATGAACCCTTTGTTTCTTGTTTATTGAGTTTGTAG

13141 GTAAAATGTATTTTTCTACATTATTGCTTATTGCTTAGTAAAATTTATTTCAT

13198 AAAACCA
13201 ACCTTTGTCATATTAGAATGTGTAGTGTTCACATGTTGCTCAGTTTTGCTAACTGATAAA
13261 TCATTTAATCCTCTTCTTCATATGTATGAGTACTATCTTATATCTGTGGTCAAGAGTGAG
13321 GTAAGCAAGCTCCAACAGACCCTGAGAACCTACGCTTGTATCCTTTCTTTGGCTAAAGAA
13381 AGCATGTCTGTTTCCTGTCAATTCTTTGAACATACAGAGTAATCTTTATAAACAAAAGAA
13441 CCTTCACCCAGCAATCAGATCGAGCAGCAACAGACAAACCAGCCAGCCAATCTCCCAAAT
13501 TTCAGGCACAAGTTTATTTTTTTTTTTTTTTATGTTTGAAAAAAGAAGATGAAGAAGAAG
13561 AAAAAAAAAAGAACAAGGAAAGATTAAACGTTAGCTTGTAAAGTTTAAAGGACCTTTCCT
13621 TTTCCTTTACGGATTTGATCAGTATGAAGTCATAAATCAAAGAAAACAGAATTGGATTTG
13681 CATTCCCAGGCGGGATGGATGCTGCCAGGAGATCACATTGCAAATAGTGAAAACAGAGGC
13741 ATTCGGTCTATGCCTGAGTCCTGTGTATAGGATCAATCTTCCTTTAATTCCGCAGTCTCC
13801 TCAGGCAATGTGACACGGGATGCAGTTTGCAGCTTTAGTGCCTTTCTTCGCCTTTTAAAT
13861 TGCCACGAATCACAGATGGCTATTTAGTGGCCCTACAATGCTGCAACACATCAGCTTGCA
13921 TTTTAGTCTTAATTATTTGTTTCTTGGATAATGGGCAGAGTTTTCTGTATTTGTATCGAC
13981 TGTTAGTGGTGAAATAGGGCTCTAGTTAACCTTTTATTTATGAAGTCTAATTTAGTGTTC
14041 CCGTGGCTAGTTGCAAGCATTTTACAGTGATCACCCAGTTTAATCTTTTGTATACTTTTT
14101 AGAAATGCCAAGAGCCTTACTAAACTGAAGCAGATTTATGATATAGTGATAATTTAGGTA
14161 GATGTTAGTCTTGAAGCTCTTATTTTGTGTGCAACTGATTATAAAAACACCTTAACCAAG
14221 TATTATTACACACATGATATCTATAACTAGGACTTTGATAACTGTTATATAAAGTGTGTA
14281 AAATTTGTATGAATAAATTTTTGTAAACAATGCAACTTGGTCTAATGTTTGGGAAAAAAG
14341 ACATTCAGGAAATAATTACTTTAAAATCTCTTAAAGTATTATATTTCTTTAGCAACCATA

FIG. 2—11

14401 AGATTTTTTTACGTCTGGAATATATATCTATCTAAGCACCCTTGTATTTTCATGAACTGC
14461 ACTTTAATAATTGATGGGCAACTGGATTCTGCTAAAAATTTAAAGTAGCTACTCAGATGG
14521 AGATGCCTAAGAAGGTTTTAAGCTCATAAACAGGCATGATGTTGCAACATTATAAGACAC
14581 ACAATTTAGATTAATTTCCATCCCCTAGTGTGTATATACTTTGCTCAATATTCAGAAAGT
14641 TACTAGGTAGTAGTGGGAGACAATGCTGGAGCATTAGTTACACATCTAAAATAGCAATCT
14701 AACATTGTTCTTTTATTTTTTATTTTAGTGGCCAGGTCTCACTATGTTGCCCAGGCTGGT
14761 CTTGCTCAAGCGATCCTCCCACCTCAGCCTCCCAAAGTGCTGGGATTACAGGCGTGAGCC
14821 ACCACACCCAGCCTAAAATAGGGATCTAACATTGTTCTTATACAAGTAACTCTGCAGACT
14881 AAACTTGTCTTGATAAAATTTTGTATAAAATGATCTAAATAATCAGTTTTTGGAGGTTTT
14941 AAAATGTATTTAGAGACATACAAACTACTGTCTCTGATTAAAATGCTTTAGGTAGAAGGA
15001 ACGTGAACATGAGTAAGTAAAGAGTTAATTAGATGCCTTTAAAAGAAAATGTACTTTGAA
15061 GTCCAGGAAGAAACACAAGAAGTCATTTGTGGATTGTATGCTTTCTTAGTTCATATTTAC
15121 AAACTTTAGGGCAAAGCTTTCATACGAAATTCCTTCAAATTCCGTAGTGGTGTGTGTTTT
15181 GGCACCTTTGACTATTTCTGGCTTAGAAAATGTATAGAAAGTCACACAATAATTGACATA
15241 CCATTTAATTTAAAATGCCAGGGTTTCATCCTAAAAATTAATGGTCTCAATTAGTAAATC
15301 AATAAATATGTTACGATAGAATTAAAGGATGAGTGAGGATTCTAAAATTATCTTCAGAAT
15361 TTAGCTCAGTATTTAAGGCCTAGAATCAAATAGGGAGGAGCCCACAGTCTAGAAATCCCG
15421 TTTGTAGTCAATGAAAAAATGAATCCAGTACAGTTCATATTTGCATTTGATTTTATTGGA
15481 TAAGGAATTTTTCTTCTCCATCTTTAACTGCCCCTCTTTGTCCTTGAAGACATAGTGTGG
15541 TAGATGAAAAAATGAAGAAGACTTTATTCGGTTGGGGCTAGGCTAATGACTTGTCAAGAA
15601 CATAAAGATAAACCCCAGACTTGGCTGACTTCAAGTGAATTTCATGTATTTAGCAACTTG
15661 CCATATTATCTTCGGTGATAACTCAAATTACATCTTTTTAAAGGCAGACTTGATACATAT
15721 GGGTATTCAAGAAGCTGTAATAGGTGCCTTAATGCTGTTAGGGCGGAGAACACACTTATT
15781 CAATACAATGCACACTTATTGAACACATGAAATGGGCCACCGCACTGGTCGAGAGAGCTT
15841 GACTAACACCTGGGAGCTCAGGTAGTATTTTTTTCAGAATGTTTTTCTGAAATGTGATCA
15901 TCTTTGGGGCGGGGGGAGCTTAAAAATGCAAAATTGTAGGGCTCTGCCAGATCCAGTGAA
15961 TCTGCATTTTAAATAAAAACCCTAGATTACTGTGCACTAAAATTTGAGACTGCCTAGATT
16021 TAGAATTGGTGACATATGTAGGCATACATATGTGCTGGTCTGAATGTTTTTTTCATATGA
16081 AATAAGCAAAAGGTCATGTTACCTGCATACAGTAATAAATACATAACTGTGCCATATTCT

FIG. 2—12

16141 TCCAAGATATCTGGTCATTAAGCTCTTTGACAATTTCAGTATTTCCTTTAGGTCACTAAA
16201 ACTACTAGTTAGCATTATTTTACTTGTACAGTCTGGTTGGACCTCTCCTACAGGAGCTTG
16261 TGGAAGGAGAGTGATCCTCTAAGTTGGGTCCAAAATATTCAATCACAGGACTAAGAGATT
16321 ATGGCTATAATGAGGAGAACTTGTGCAGCTAGCTAGCCATAATTCTGGGGATCCAGAAGT
16381 CAACTTCCAGTTGCATTATATCCCAATTTGGTTTGAATGTATTTACTGCTCCCCAACTGT
16441 TTACATGATGGTTTCTCTTGGATGGCTCACTATGACCTTCAACCCAACCCTACTGTTCAC
16501 ATGATCACAAGATTGGAAGCCAAGATCAAGTCATCCCTCTTCTCTTTTGTTGCCACTCTT
16561 TTTTGTAGAAGGGAGATGCCAGCTGCCCCTGCTGCTGCAGATTGCATCACTGCTGGATTC
16621 TTACATTGGTTTGTAGTTGGTCATCCTGGTCACTTCCCCTGCAACCACATAGTTTTAGCT
16681 CCATCTTAGTATCATGTCCCTCTGATCAGATGTTCCAGAGTAGCTTCCATTGTCAAAGGG
16741 TTAAAGGGTTTAAGGTAATCAGTAGTCAATTTTACCTCTCTGTTCTTCAACACGATCCTT
16801 CCTCTTTTTTTTGTTTTGAGAAAGGGTCTTACTCTGTTGCCCAGGCTGGAGTGCAGTGGC
16861 ACGATCTCGGCTCACTGCAACCTCTGCCTCCCGGGTTTAAGCGATTCTCCTGTCTCAACC
16921 TCCCGAGTAGCTGGGATTACAGGTGCATGCCAACGCGCCCGGCTAATTTTTGTATTTTTA
16981 GTAGAGACGGGGTTTCACTGTGTTGGCCAGGCTGGTCTTGGACTCTTGTCCCCAAATGAT
17041 C